# EUROPEAN PATENT SPECIFICATION

(11) **EP 3 095 864 B1**
(45) Date of publication and mention of the grant of the patent: **15.04.2020**
(21) Application number: 15737214.5
(22) Date of filing: 14.01.2015
(51) Int. Cl.: C12N 15/867, C12N 15/09, C12N 5/10, C12Q 1/02, C12Q 1/04, C12Q 1/68, A61K 48/00, G01N 33/50

(54) **METHOD FOR LABELLING CANCERIZATION-CAUSING CELL IN STEM CELLS**
VERFAHREN ZUR MARKIERUNG EINER KANZERISIERUNGSVERURSACHENDEN ZELLE IN STAMMZELLEN
PROCÉDÉ D'ÉTIQUETAGE D'UNE CELLULE RESPONSABLE DE LA CANCÉRISATION DANS DES CELLULES SOUCHES

(30) Priority: 14.01.2014 JP 2014004262
(43) Date of publication of application: 23.11.2016
(60) Divisional application: 20159605.3
(73) Proprietor: Kagoshima University, Kagoshima-shi, Kagoshima 890-8580 (JP)
(72) Inventor: KOSAI, Ken-ichiro, Kagoshima-shi Kagoshima 890-8580 (JP); MITSUI, Kaoru, Kagoshima-shi Kagoshima 890-8580 (JP); IDE, Kanako, Kagoshima-shi Kagoshima 890-8580 (JP)
(74) Representative: J A Kemp LLP
(86) International application number: PCT/JP2015/000138
(87) International publication number: WO 2015/107888

(56) References cited:
- WO-A1-2012/133674
- JP-A- 2005 095 027
- JP-A- 2008 541 768
- JP-A- 2013 530 699
- SONG-TAO YU ET AL: "Noninvasive and real-time monitoring of the therapeutic response of tumors in vivo with an optimized hTERT promoter", CANCER, vol. 118, no. 7, 1 April 2012 (2012-04-01) , pages 1884-1893, XP055213274, ISSN: 0008-543X, DOI: 10.1002/cncr.26476
- TIONG-TI LIM ET AL: "Lentiviral Vector Mediated Thymidine Kinase Expression in Pluripotent Stem Cells Enables Removal of Tumorigenic Cells", PLOS ONE, vol. 8, no. 7, 30 July 2013 (2013-07-30), page e70543, XP055396844, DOI: 10.1371/journal.pone.0070543
- FUYI CHENG ET AL: "Protecting against wayward human induced pluripotent stem cells with a suicide gene", BIOMATERIALS, ELSEVIER SCIENCE PUBLISHERS BV., BARKING, GB, vol. 33, no. 11, 9 January 2012 (2012-01-09), pages 3195-3204, XP028414062, ISSN: 0142-9612, DOI: 10.1016/J.BIOMATERIALS.2012.01.023 [retrieved on 2012-01-11]
- BARESE CECILIA ET AL: "Suicide Gene (HSVtk) Ablation of Hematopoietic Stem Cells and Their Progeny In the Rhesus Macaque Model: An Approach to Deletion of Dangerous Clones", BLOOD, vol. 116, no. 21, November 2010 (2010-11), pages 1540-1541, XP008185330, & 52ND ANNUAL MEETING OF THE AMERICAN-SOCIETY-OF-HEMATOLOGY (ASH); ORLANDO, FL, USA; DECEMBER 04 -07, 2010
- FEHSE B ET AL: "A NOVEL 'SORT-SUICIDE' FUSION GENE VECTOR FOR T CELL MANIPULATION", GENE THE, NATURE PUBLISHING GROUP, GB, vol. 9, no. 23, 1 December 2002 (2002-12-01), pages 1633-1638, XP009010172, ISSN: 0969-7128, DOI: 10.1038/SJ.GT.3301828
- GRIFFIOEN MARIEKE ET AL: "Retroviral gene transfer of T cell receptors (TCR) specific for minor histocompatibility antigens to virus-specific T cells as cellular immunotherapy of patients with relapsed hematological malignancies after allogeneic stem cell transplantation.", November 2005 (2005-11), BLOOD, VOL. 106, NR. 11, PART 2, PAGE(S) 470B-471B, 47TH ANNUAL MEETING OF THE AMERICAN-SOCIETY-OF-HEMATOLOGY; ATLANTA, GA, USA; DECEMBER 10 -13, 2005, XP008185332, ISSN: 0006-4971 * the whole document * * page 2 *
- NAUJOK ORTWIN ET AL: "Selective Removal of Undifferentiated Embryonic Stem Cells from Differentiation Cultures Through HSV1 Thymidine Kinase and Ganciclovir Treatment", STEM CELL REVIEWS AND REPORTS, vol. 6, no. 3, October 2010 (2010-10), pages 450-461, XP008185395,
- FEI CHEN ET AL: "Suicide gene-mediated ablation of tumor-initiating mouse pluripotent stem cells", BIOMATERIALS., vol. 34, no. 6, 1 February 2013 (2013-02-01), pages 1701-1711, XP55394805, GB ISSN: 0142-9612, DOI: 10.1016/j.biomaterials.2012.11.018
- Kanako, I. et al.: "An efficient construction of vectors that identify and eliminate tumorigenic cells in pluripotent stem cells", , 1 May 2016 (2016-05-01), XP55394779, Retrieved from the Internet: URL:http://www.sciencedirect.com/science/a rticle/pii/S1525001616330507/pdfft?md5=f61 a74b54c812ad7e0d66d289d2a9ef8&pid=1-s2.0-S 1525001616330507-main.pdf [retrieved on 2017-07-31]
- KOSAI KEN-ICHIRO ET AL: "A Novel Construction of Lentiviral Vectors for Eliminating Tumorigenic Pluripotent Stem Cells", MOLECULAR THERAPY, ACADEMIC PRESS, US, vol. 25, no. 5, Supplement 1, 1 May 2017 (2017-05-01), page 70, XP008185320, ISSN: 1525-0016, DOI: 10.1016/J.YMTHE.2017.04.025
- KAORU MITSUI ET AL: "Viral Vector-Based Innovative Approaches to Directly Abolishing Tumorigenic Pluripotent Stem Cells for Safer Regenerative Medicine", MOLECULAR THERAPY - METHODS AND CLINICAL DEVELOPMENT, vol. 5, 1 June 2017 (2017-06-01), pages 51-58, XP055394785, GB ISSN: 2329-0501, DOI: 10.1016/j.omtm.2017.03.002
- SONG-TAO YU ET AL.: 'Noninvasive and real-time monitoring of the therapeutic response of tumors in vivo with an optimized hTERT promoter' CANCER, [Online] vol. 118, no. ISSUE., 25 August 2011, pages 1884 - 1893, XP055213274 Retrieved from the Internet: <URL:http://onlinelibrary. wiley .com/doi/10.1002/ cncr.26476/full>
- Kaoru Mitsui ET AL: "Conditionally replicating adenovirus prevents pluripotent stem cell-derived teratoma by specifically eliminating undifferentiated cells", MOLECULAR THERAPY - METHODS & CLINICAL DEVELOP, vol. 2, 1 January 2015 (2015-01-01), page 15026, XP55394780, GB ISSN: 2329-0501, DOI: 10.1038/mtm.2015.26
- TAKAHASHI ET AL: "Identification and Isolation of Embryonic Stem Cell-Derived Target Cells by Adenoviral Conditional Targeting", MOLECULAR THERAPY : THE JOURNAL OF THE AMERICAN SOCIETY OF GENE THERAPY, ACADEMIC PRESS ; NATURE PUBLISHING GROUP, US, vol. 14, no. 5, 9 October 2006 (2006-10-09), pages 673-683, XP005844850, ISSN: 1525-0016, DOI: 10.1016/J.YMTHE.2006.05.010
- Konala, V. B. R. et al.: "Embryonic stem cells: development and characterization" In: "Applications of flow cytometry in stem cell research and tissue regeneration.", 2010, Krishan, A. et al., USA, XP009512069, ISBN: 978-0-470-54398-6 DOI: 10.1002/9780470631119.ch10,
- LI XIAJUN ET AL: "Identifying genes preferentially expressed in undifferentiated embryonic stem cells", BMC CELL BIOLOGY, BIOMED CENTRAL, LONDON, GB, vol. 8, no. 1, 28 August 2007 (2007-08-28) , page 37, XP021027713, ISSN: 1471-2121, DOI: 10.1186/1471-2121-8-37

### SEQUENCE LISTING

<110> Kagoshima University
<120> Labeling and treatment method of tumorigenesis-causing cells in stem cells
<130> PW14010KG
<150> JP2014-004262
   <151> 2014-01-14
<160> 18
<170> PatentIn version 3.5
<210> 1
   <211> 28
   <212> DNA
   <213> Artificial Sequence
<220>
   <223> primer
<400> 1
   accggtcacc atggtgaggg agctgatt 28
<210> 2
   <211> 28
   <212> DNA
   <213> Artificial Sequence
<220>
   <223> primer
<400> 2
   agatcttctg tgccccagtt tgctaggg 28
<210> 3
   <211> 30
   <212> DNA
   <213> Artificial Sequence
<220>
   <223> primer
<400> 3
   accggtcacc atggtgagca agggcgagga 30
<210> 4
   <211> 30
   <212> DNA
   <213> Artificial Sequence
<220>
   <223> primer
<400> 4
   ggatcccttg tacagctcgt ccatgccgag 30
<210> 5
   <211> 30
   <212> DNA
   <213> Artificial Sequence
<220>
   <223> primer
<400> 5
   ggatccatgg cttcgtaccc cggccatcaa 30
<210> 6
   <211> 30
   <212> DNA
   <213> Artificial Sequence
<220>
   <223> primer
<400> 6
   gtttaaactc agttagcctc ccccatctcc 30
<210> 7
   <211> 30
   <212> DNA
   <213> Artificial Sequence
<220>
   <223> primer
<400> 7
   ggatccatgg cggcccggcg cggggctctc 30
<210> 8
   <211> 29
   <212> DNA
   <213> Artificial Sequence
<220>
   <223> primer
<400> 8
   cccgggtacc tcacttccat agctccccc 29
<210> 9
   <211> 35
   <212> DNA
   <213> Artificial Sequence
<220>
   <223> primer
<400> 9
   ttctacgcgt accggtgcca tgctcgaggg agtgc 35
<210> 10
   <211> 22
   <212> DNA
   <213> Artificial Sequence
<220>
   <223> primer
<400> 10
   gggcccggga ttttcctcca cg 22
<210> 11
   <211> 35
   <212> DNA
   <213> Artificial Sequence
<220>
   <223> primer
<400> 11
   gaaaatcccg ggcccatggt gagcaagggc gagga 35
<210> 12
   <211> 35
   <212> DNA
   <213> Artificial Sequence
<220>
   <223> primer
<400> 12
   tggtcttaaa ggtaccttac ttgtacagct cgtcc 35
<210> 13
   <211> 36
   <212> DNA
   <213> Artificial Sequence
<220>
   <223> primer
<400> 13
   gaaaatcccg ggcccatggt gagcgagctg attaag 36
<210> 14
   <211> 36
   <212> DNA
   <213> Artificial Sequence
<220>
   <223> primer
<400> 14
   tggtcttaaa ggtacctcat ctgtgcccca gtttgc 36
<210> 15
   <211> 26
   <212> DNA
   <213> Artificial Sequence
<220>
   <223> primer
<400> 15
   cacctaaagt gaaatgaggt aaagcc 26
<210> 16
   <211> 22
   <212> DNA
   <213> Artificial Sequence
<220>
   <223> primer
<400> 16
   ggaaagatca tagaaagaag ag 22
<210> 17
   <211> 25
   <212> DNA
   <213> Artificial Sequence
<220>
   <223> primer
<400> 17
   caccgaattc taccgggtag gggag 25
<210> 18
   <211> 20
   <212> DNA
   <213> Artificial Sequence
<220>
   <223> primer
<400> 18
   ctgcaggtcg aaaggcccgg 20

## Description

### TECHNICAL FIELD

The present invention relates to a method for searching for a promoter for use in labeling and removal of tumorigenic cells remaining after differentiation of stem cells.

### BACKGROUND ART

Human embryonic stem cells (ES cells) and human induced pluripotent stem cells (iPS cells) are expected to serve as base tools for medical and pharmaceutical creation such as: (1) human cells used in a drug toxicity test and the like for drug discovery and development; (2) disease model cells used for *in vitro* elucidation of disease induction/pathological conditions; and (3) cells for transplantation used for regenerative medicine. For most anticipated regenerative medicine, clinical trials in human patients using human ES cells have already been initiated for some diseases in the United States. In Japan, clinical studies using human iPS cells for ocular diseases have been initiated.

At present, the greatest problem in clinical application of human ES/iPS cells is mixing of remaining undifferentiated cells and desired differentiated cells. There is a report that teratoma is formed with undifferentiated cells remaining after transplantation of cells subjected to differentiation induction treatment (Non-Patent Document 1). Teratoma is said to be benign tumor; however, it is highly likely to cause various side effects. For example, if cells other than cardiomyocytes are transplanted into the heart, it would possibly result in serious side effects such as lethal arrhythmia.

There is another report that iPS cells that have been artificially modified by initializing differentiated cells are highly likely to generate not only teratoma but also malignant tumor (Non-Patent Document 2). This is probably because activation of c-Myc that is one of the genes required for establishment of iPS cells and also known as a carcinogenic gene causes malignant transformation of cells, and the gene is introduced into somatic cells via retrovirus or lentivirus upon establishment of iPS cells, which may cause destruction of genes that are important for maintaining normal functions of cells (e.g., cancer suppressor genes) or abnormal activation of genes concerning cell growth.

Therefore, a method for obtaining purified differentiated cells, the method comprising efficiently and comprehensively removing undifferentiated cells remaining among cells subjected to differentiation treatment has been awaited. To date, a method for establishing iPS cells using three factors other than c-Myc (Non-Patent Documents 3 and 4) and a method for suppressing damage to chromosomes by establishing iPS cells using plasmids instead of viral vectors (Non-Patent Documents 5 and 6) have been reported. Although these methods are expected to bring about certain effects of suppressing "tumorigenesis" of cells, they are not intended to prevent the presence of remaining undifferentiated cells or remove undifferentiated cells that have retrodifferentiated after transplantation, for example, which have been problematic.

As another approach, a method for selecting and using a clone that is unlikely to cause tumorigenesis after differentiation has been reported (Patent Document 1). However, also in this case, the method is not intended to prevent the presence of remaining undifferentiated cells or completely suppress tumorigenesis.

Also, a method for killing remaining undifferentiated cells as target cells has been studied. For example, it has been attempted to introduce a lentiviral vector carrying the herpes simplex virus thymidine kinase (HSV-tk) gene downstream of the Nanog promoter into mouse ES/iPS cells or human ES/iPS cells, thereby causing these cells to have ganciclovir (GCV) sensitivity. It has been reported that when such cells are transplanted subcutaneously in immunodeficient mice for tumorigenesis, tumors can be reduced by GCV administration (Non-Patent Documents 7 and 8).

Regarding suicide gene therapy with the use of the above combination of HSV-tk and GCV, some studies on techniques of removing tumorigenic stem cells in regenerative medicine, have been published at the present stage. Therefore, there is no case of the clinical application of such techniques for regenerative medicine of stem cells. On the other hand, there is a long history of research on the applied use of HSV-tk/GCV for cancer gene therapy. HSV-tk/GCV gene therapy (typically including administering a viral vector for HSV-tk gene transfer/expression directly into tumors of a patient and injecting GCV to the patient) has been performed in many clinical trials for cancer patients. If HSV-tk, which is a virus-derived gene but not a human-derived gene, is introduced into cells, it might result in induction of immunity against heterologous proteins. In cancer therapy, if cancer cells transfected with the HSV-tk gene are removed due to induction of immunity mainly involving cellular immunity, it leads to amplification of therapeutic effects. Therefore, it would be rather advantageous that the HSV-tk gene is a virus-derived gene. However, in regenerative medicine, if the HSV-tk gene is introduced into cells for transplantation, transplanted cells might be eliminated as a result of induction of immunity against heterologous proteins. In this regard, it would be more desirable to use human-derived genes. Another reported strategy is a technique of inducing the p53 and p16 genes that function to inhibit a cell growth cycle by doxycycline and using the genes (Patent Document 2). However, it is impossible for such technique to kill all tumorigenic cells because tumor suppressor genes are not originally intended to actively or directly kill tumor cells. Therefore, the technique has been significantly problematic in terms of efficacy and usefulness. This is because there is a risk that remaining viable undifferentiated cells start to proliferate after the cancellation of doxycycline induction, and there is another risk that a so-called doxycycline gene expression induction system with low specificity results in non-specific expression of the above genes, which might influence differentiation induction of stem cells, biological dynamics, and the like.

Meanwhile, as a method for distinguishing undifferentiated cells, the following have been disclosed: a method for distinguish undifferentiated cells based on the abundance of SP1 (Specificity Protein 1), proteins contained in Cajal bodies, proteins contained in the nuclear lamina, proteins contained in a perinucleolus compartment, a protein contained in the PML body, or the like (Patent Document 3); a method for detecting transformed cells contained in a cell population by examining the expression or non-expression of mRNA of the hTERT or IPAS gene (Patent Document 4); and a method for identifying cells expressing the podocalyxin-liked protein (PODXL) on their surfaces (Patent Document 5). However, each of these methods requires a complicate operation, which makes it impossible to conveniently identify, for example, remaining undifferentiated cells in a tissue transplanted in the living body.

It has been attempted to distinguish undifferentiated cells based on its promoter activity. A method using the Stm1 promoter sequence has been disclosed (Patent Document 6).

Previously, the present inventors provided a method for selectively killing remaining undifferentiated cells using a "Conditionally replicating adenovirus that can target cancers with multiple cancer-specific factors" (m-CRA) (Patent Document 7). The method is very useful to solve the above problem because tumorigenic cells and undifferentiated cells are actively and directly killed, and cell killing effects are exerted on non-transfected tumorigenic cells. Meanwhile, according to the method, a target gene is not introduced into all stem cells in advance. Accordingly, there was no guarantee whether the induction of immunity against the "Conditionally replicating adenovirus that can target cancers with multiple cancer-specific factors" (m-CRA) can be completely cancelled *in vivo* if tumorigenic cells are generated from stem cells to which the virus has not reached.

Non-Patent Document 9 discloses noninvasive and real-time monitoring of the therapeutic respone of tumors *in vivo* with an optimized hTERT promoter. Non-Patent Document 10 discloses suicide gene (HSVtk) ablation of hematopoietic stem cells and their progeny in a rhesus macaque model. Non-Patent Document 11 discloses a "sort-suicide" fusion gene vector for T cell manipulation. Non-Patent Document 12 discloses that lentiviral vector mediated thymidine kinase expression in pluripotent stem cells enables removal of tumorigenic cells. Non-Patent Document 13 discloses that conditionally replicating adenovirus prevent pluripotent stem-cell derived teratoma formation by specifically eliminating undifferentiated cells. Non-Patent Document 14 discloses identification and isolation of embryonic stem-cell derived target cells by adenoviral conditional targeting. Non-Patent document 15 discloses an in-vitro method for screening for an undifferentiated-cell-specific gene, and thereby indirectly its promoter by making use of an retroviral gene trap vector to target genes expressed in undifferentiated ES cells.

### PRIOR ART REFERENCES

### PATENT DOCUMENTS

Patent Document 1: WO2012/115270
Patent Document 2: Japanese Unexamined Patent Publication (Kokai) No. 2013-9742
Patent Document 3: Japanese Unexamined Patent Publication (Kokai) No. 2012-223104
Patent Document 4: Japanese Unexamined Patent Publication (Kokai) No. 2012-072063
Patent Document 5: Japanese Unexamined Patent Publication (Kohyo) No. 2009-528838
Patent Document 6: Japanese Unexamined Patent Publication (Kokai) No. 2005-095027
Patent Document 7: WO2012/133674

### NON-PATENT DOCUMENTS

Non-Patent Document 1: TAKAHASHI, K. et al. (2007), Cell 131: 861-72
Non-Patent Document 2: MIURA, K. et al. (2009), Nature Biotechnology 27:743-745
Non-Patent Document 3: NAKAGAWA, M. et al. (2008), Nat. Biotechnol. 26: 101-6
Non-Patent Document 4: WERNIG, M. et al. (2008), Cell Stem Cell 2: 10-2
Non-Patent Document 5: OKITA, K. et al. (2008), Science 7: 949-53
Non-Patent Document 6: YU, J. et al. (2009), Science 8: 797-801
Non-Patent Document 7: FUYI, CHENG, et al. (2012), Biomaterials 33: 3195-204
Non-Patent Document 8: FEI, CHEN et al. (2013), Biomaterials 34: 1701-11
Non-Patent Document 9: YU, S. et al. (2011), Cancer 118.7: 1884-1893.
Non-Patent Document 10: BARESE, C. et al. (2010), Blood 116.21: 1540-1541
Non-Patent Document 11: FEHSE, B. et al. (2002), Gene therapy 9.23: 1633-1638.
Non-Patent Document 12: LIM, T. et al. (2013), PLoS One 8.7: e70543.
Non-Patent Document 13: MITSUI, K. et al. (2015), Molecular Therapy-Methods & Clinical Development 2: 15026.
Non-Patent Document 14: TAKAHASHI, T. et al. (2006), Molecular Therapy 14.5: 673-683.
Non-Patent Document 15: Li, X. & Leder, P. (2007) BMC Cell Biology, 8.37, 1-14.

### PROBLEMS TO BE SOLVED

The conventional methods for labeling and removing tumorigenic cells remaining after differentiation of stem cells have been problematic in terms of efficacy, convenience, and the like. A feasible method for identifying and removing remaining undifferentiated cells/tumorigenic cells has been under development. In order to label/remove tumorigenic cells remaining after differentiation of stem cells, technologies including the following are necessary: vectors that can efficiently introduce foreign genes into all undifferentiated cells without inhibiting genes maintaining normal cell functions; efficient search/identification and demonstration of promoters that can function in a manner specific to undifferentiated cells; and suicide genes that selectively visualize/identify undifferentiated cells and securely kill tumorigenic cells in a selective manner when needed. Eventually, a vector system containing all of these elements and a technique for conveniently and efficiently creating various candidate vectors in the system are necessary. To date, no technology satisfying all of these requirements has been found.

Labeling and removal of tumorigenic cells remaining differentiation of stem cells have so far been carried out separately. According to the conventional methods, after labeling tumorigenic cells, they must be removed using different techniques. There has been a problem that complicated operations for identifying and removing tumorigenic cells are necessary. In addition, since the cells are identified and removed by completely independent and different techniques and processes, it was not always guaranteed that labeled tumorigenic cells could be completely removed by different techniques of removal. In particular, there is no progress in research on techniques for securely removing and killing tumorigenic cells and remaining undifferentiated cells. Thus, no effective means has been available.

The object of the present invention is to provide a method for searching for the best possible promoter to securely target tumorigenic cells remaining after differentiation of stem cells.

### MEANS FOR SOLVING THE PROBLEMS

As a result of intensive studies on gene transfer technology for efficiently and comprehensively labeling/removing undifferentiated cells remaining after differentiation induction treatment, the present inventors found, as a basic backbone vector, a viral vector containing an expression cassette, in which a marker gene and a toxic gene are bound via a sequence capable of allowing a promoter to cause the two genes to be simultaneously expressed downstream of the promoter region in which recombination is possible using a recombination system. This vector, in which a marker gene and a toxic gene are bound via a sequence capable of allowing a promoter to cause the two genes to be simultaneously expressed, is able to impart both functions of labeling and cell killing at one instance of gene transfer. Further, since the vector can conveniently incorporate an arbitrary promoter using a recombination system, a promoter to be used can be appropriately selected depending on, for example, depending on cells of interest that are induced to differentiate and the timing of cell removal (before or after transplantation). Furthermore, it is possible to use the vector in order to conveniently analyze whether a promoter has activity specific to undifferentiated cells or tumorigenic cells. Moreover, by adopting a drug-dependent suicide gene as a toxic gene for the vector , it is possible to configure the vector not to exhibit cell killing effects without drug-dependent induction as well as cell selectivity of tumor-specific/undifferentiated-cell-specific promoters. That is, when the vector is merely introduced into stem cells, the vector does not kill the cells. Therefore, the vector can be introduced into all stem cells before differentiation induction treatment, thereby preventing a failure in gene transfer. Accordingly, double safety consisting of cell selectivity of tumor-specific/undifferentiated-cell-specific promoters and drug dependence can be ensured. In addition, by making use of a toxic gene providing a killing mechanism specific to (drug-dependent) proliferating cells specific in the vector, it is possible to impart proliferating cell selectivity of the killing mechanism as well as cell selectivity of tumor-specific/undifferentiated-cell-specific promoters and drug dependence. Thus, triple specificity/safety can be ensured.

The present inventors found that immunogenicity issues can be resolved using, as a drug-dependent suicide gene carried by the vector described herein, human-tmpk and iCaspase9 which are human-derived enzymes. Further, the present inventors searched for a promoter having activity specific to undifferentiated cells or tumorigenic cells, which is carried by the vector described herein. Surprisingly, it was found that the survivin promoter, which is generally known as a tumor (cancer)-specific promoter, shows unexpectedly high activity in normal undifferentiated human ES/iPS cells.

Furthermore, the present inventors found a method for efficiently and comprehensively labeling/removing undifferentiated cells remaining after differentiation induction treatment using the vector described herein and a method for conveniently analyzing whether or not a promoter has activity specific to undifferentiated cells or tumorigenic cells.

The present invention provides:
(1) An *in vitro* method for screening for an undifferentiated-cell-specific promoter, the method comprising the steps of:
   A) replacing the promoter of a lentiviral vector containing a nucleic acid sequence in which a fluorescent protein gene and a drug-dependent suicide gene are bound via a sequence capable of allowing a promoter to express the two genes simultaneously and containing a recombination cassette having the promoter region between sequences recognized by site-specific recombinases, wherein the promoter region is operably linked to the fluorescent protein gene and the drug-dependent suicide gene by a test promoter to prepare a viral vector containing the test promoter;
   B) infecting cells of interest with the lentiviral vector containing the test promoter;
   C) detecting the expression of the fluorescent protein gene in said infected cells subjected to differentiation induction treatment;
   D) distinguishing whether said infected cells subjected to differentiation induction treatment are undifferentiated cells or differentiated cells; and
   E) judging whether or not the test promoter is specific to undifferentiated cells,
      wherein if the expression level of the fluorescent protein gene in undifferentiated cells is higher than in differentiated cells with a statistical difference, the test promoter is determined to be specific to undifferentiated cells.
(2) The method according to (1), wherein the sequence capable of allowing a promoter to cause the two genes to be simultaneously expressed is 2A sequence.
(3) The method according to (1) or (2), wherein the drug-dependent suicide gene is HSV-tk, human-tmpk, a cytosine deaminase gene, or caspase.
(4) The method according to (3), wherein the drug-dependent suicide gene is HSV-tk.
(5) The method according to any one of (1)-(4), wherein the fluorescent protein is a red fluorescent protein or green fluorescent protein.
(6) The method according to (5), wherein the fluorescent protein is mKate2 or Venus.

The term "stem cells" used herein refers to cells having pluripotency and replication competence. Therefore, the term "stem cells" used herein encompasses stem cells, ES cells, iPS cells, neural stem cells, hematopoietic stem cells, mesenchymal stem cells, liver stem cells, pancreatic stem cells, skin stem cells, muscle stem cells, or germlinestem cells. The term "stem cells" used herein refers to cells having pluripotency and more preferably embryonic stem cells (ES cells) and induced pluripotent stem cells (iPS cells). It is possible to confirm whether certain cells are stem cells based on the prerequisite that stem cells are cells that form an embryoid body in an *ex vivo* culture system or cells that differentiate into desired cells after culture under differentiation induction conditions (differentiation treatment), for example. It is also possible to confirm whether certain cells are stem cells based on the prerequisite that stem cells are cells that form teratoma when transplanted in immunodeficient mice using a living body, cells that form chimeric embryo when injected into a blastocyst, and cells that proliferate when transplanted into tissue or injected into ascites in a living body. Alternatively, it is possible to confirm whether certain cells are stem cells based on the prerequisite that stem cells are: cells positive to alkaline phosphatase staining, SSEA3 staining, SSEA4 staining, TRA-1-60 staining, and/or TRA-1-81 staining; cells expressing the oct3/4, nanog, sox2, cripto, dax1, eras, fgf4, esg1, rex1, zfp296, utf1, gdf3, sall4, tbx3, tcf3, dnmt31, and /or dnmt3b genes; and cells expressing miR-290 and/or miR-302. It is also possible to confirm whether certain cells are stem cells based on the prerequisite that stem cells are, for example, cells in which telomerase reverse transcriptase or survivin is highly expressed.

The term "differentiation" used herein refers to a phenomenon in which daughter cells having specific functional or morphological features are generated as a result of division of pluripotent cells. The terms "differentiation treatment" and "differentiation induction treatment" used herein have the same meaning and refer to a treatment that induces stem cells into differentiated cells. It has been reported that cell differentiation is induced by a variety of methods. It is possible to induce pluripotent cells to differentiate by differentiation induction treatment using differentiation inducers or the like depending on types of cells that are induced to differentiate and the like. The term "differentiated cells" refers to daughter cells having specific functional or morphological features, which are generated as a result of differentiation. In general, differentiated cells are stable and have low proliferative capacity. Aside from exceptions, they do not differentiate into a different type of cells.

The term "undifferentiated cells" refers to undifferentiated cells, including undifferentiated cells during differentiation and imperfectly differentiated cells. In particular, the term "undifferentiated cells" used herein is interpreted as cells that have not differentiated after differentiation induction treatment unless there is inconsistency in such interpretation. Undifferentiated cells used herein do not necessarily have perfect features of stem cells described above and have the proliferative (self-proliferative) ability greater than (for example, the growth rate two times, three times, five times, or 10 times or more than) that of differentiated cells. Particularly preferably, "undifferentiated cells" are "tumorigenic cells." It is possible to determine whether or not certain cells are undifferentiated cells by, for example, judging cells in which c-Myc is activated or cells in which telomerase reverse transcriptase and/or survivin are highly expressed as cells having the tumorigenic ability.

The term "tumorigenic cells" used herein refers to undifferentiated cells that remain undifferentiated even after differentiation treatment of stem cells and are able to generate tumor cells. Tumorigenic cells encompass not only cells maintaining properties comparable to those of stem cells before differentiation but also cells having properties different from those of stem cells before differentiation but remaining undifferentiated, such as oligopotent cells (cells that can differentiate into only several types of cells) and cells having the tumorigenic ability (e.g., cancer stem cells). Here, the expression "able to generate tumor cells" does not necessarily mean that cells are able to generate tumor cells with a probability of 100%; however, it means that cells have the ability to generate tumor cells greater than that of differentiated cells. It is possible to judge whether or not certain cells have the tumorigenic ability by judging, for example, cells in which c-Myc is activated or cells in which telomerase reverse transcriptase and/or survivin are highly expressed as cells having the tumorigenic ability. Undifferentiated cells, which are expressed herein as "undifferentiated cells remaining after differentiation treatment," "remaining undifferentiated cells," "undifferentiated cells among cells subjected to differentiation treatment," or the like, and especially undifferentiated cells remaining after differentiation induction treatment of stem cells may be "tumorigenic cells."

The term "marker gene" used herein refers to a gene encoding a labeling substance that allows cells transfected with the gene to be detected or assayed. In the invention, the marker gene is a fluorescent protein gene. Examples of marker genes that are fluorescent protein genes include: genes encoding red, green, blue, and yellow fluorescent proteins such as green fluorescent protein (GFP), EGFP (enhanced GFP) or Venus that is obtained by changing the amino acid sequence of GFP to improve the labeling ability, red fluorescent protein (RFP) that has a different fluorescence wavelength, blue fluorescent protein (BFP), yellow fluorescent protein (YFP), and mKate2. Preferably, mKate2 or Venus is used. The expression "labeling" used herein means to identify the presence or location of cells of interest temporarily or for a long term depending on the intended use so as to determine the number (or the amount or proportion) of cells of interest, thereby making it possible to distinguish the cells of interest from other cells. In addition, the expression "identifying" used herein means to identify the presence or location of cells of interest temporarily or for a long term, and the expression "distinguishing" means to distinguish cells of interest from other cells or tissue temporarily or for a long term. Therefore, labeling described herein is not particularly limited as long as the presence or location of cells of interest can be identified or cells of interest can be distinguished from other cells, and labeling does not require cells other than cells of interest not to be stained. For example, when the presence of undifferentiated cells among cells subjected to differentiation treatment is distinguished based on the expression of a marker gene contained in the vector of the present disclosure, staining may be carried out to an extent that allows undifferentiated cells to be distinguished when compared with differentiated cells among the cells. This does not prevent differentiated cells from being stained (weakly).

The term "toxic gene" used herein refers to a gene having the ability to eventually kill transfected cells. In the invention, the toxic gene is a drug-dependent suicide gene. The term "drug-dependent suicide gene" refers to a gene encoding a protein that converts a non-toxic prodrug (drug) to a toxic substance or a gene encoding a non-toxic protein that is converted to a toxic substance with the addition of a drug. Also the expression "drug that allows a drug-dependent suicide gene to exhibit toxicity" means a drug that causes toxicity by interacting with the drug-dependent suicide gene. Specifically, the drug-dependent suicide gene may be a gene encoding a metabolic enzyme protein (prodrug convertase) that converts a non-toxic prodrug (drug) to a toxic substance. In this case, the expression "drug that allows a drug-dependent suicide gene to exhibit toxicity" refers to a non-toxic prodrug which is converted to a toxic substance by the drug-dependent suicide gene (metabolic enzyme).. The term "drug-dependent suicide gene" used herein may be interpreted as "inducible toxic gene" unless there is inconsistency in such interpretation.

Examples of a combination of a drug-dependent suicide gene and a prodrug (drug) include: herpes simplex virus thymidine kinase and ganciclovir or acyclovir; VaricellaZoster virus thymidine kinase and 6-methoxypurine arabinonucleoside (Huber et al., 1991, Proc. Natl. Acad. Sci. USA 88: 8039); *E.coli* cytosine deaminase and fluorouracil (Mullen et al., 1992, Proc. Natl. Acad. Sci. USA. 89: 33); *E. coli* xanthine-guanine phosphoribosyltransferase and thioxanthine (Beshard et al., 1987, Mol. Cell Biol. 7: 4139); *E. coli* or *Leishmania* purine nucleoside phosphorylase and non-toxic purine deoxyadenosine, adenosine, deoxyguanosine, or guanosine derivative (Koszalka and Krenitsky, 1979, J. Biol Chem 254: 8185, 1979; Sorscher et al., 1994, GeneTherapy 1: 233); cytochrome pla50 2B1 or cytochrome p450 reductase and 3-amino-1,2,4-benzotriazin-1,4-dioxide (Walton et al., 1992, Biochem. Pharmacol. 44: 251)); cell surface alkaline phosphatase and etoposide monophosphate (Senter et al., 1988, Proc. Natl. Acad. Sci. USA 85: 4842, 1988); nitroreductase and metronidazole or nitrofurantoin (Hof et al., 1988, Immunitat undInfektion 16: 220); N-deoxyribosyl transferase and 1-deazapurine (Betbeder et al., 1989, NucleicAcids Res 17: 4217); pyruvate ferredoxin oxidoreductase and metronidazole (Upcroft et al., 1990, Int. J. Parasitolog, 20: 489); carboxypeptidase G2 and amino acylate nitrogen mustard (Antoniw et al., 1990, Brit J. Cancer 62: 909); carboxypeptidase A and methotrexate α alanine (Haenseler et al., 1992, Biochemistry 31: 891); lactamase and a cephalosporin derivative (Meyer et al., 1993, CancerRes. 53: 3956; and Vradhula et al., 1993, Bioconiugate Chemistry 4: 334); an actinomycin D synthetase complex and a synthetic pentapeptidelactone precursor (Katz et al., 1990, J. Antibiotics 43: 231); and glucuronidase and a glucuronide derivative of a toxic substance such as doxorubicin (Bosslet et al., 1994, Cancer Res. 54: 2151; Haeberlin et al., 1993, Pharmaceutical Res. 10: 1553).

For example, when a thymidine kinase gene such as HSV-tk is used as a drug-dependent suicide gene and ganciclovir (GCV) is used as a drug, GCV is metabolized by HSV-tk to form ganciclovir triphosphate that is a toxic substance. Also, when human-tmpk is used as a drug-dependent suicide gene and azidothymidine (AZT) is used as a non-toxic prodrug, AZT is metabolized by human-tmpk to form AZT triphosphate that is a toxic substance. In addition, when cytosine deaminase (CD) from a bacterium such as *Escherichia coli* is used as a drug-dependent suicide gene and 5-fluorocytosine (5-FC) is used as a prodrug, 5-FC is metabolized by CD to form 5-fluorouracil (5-FU) that is a toxic substance. When herpes virus thymidine kinase is used as a drug-dependent suicide gene and 6-methoxypurine arabinonucleoside (Ara-M) is used as a non-toxic prodrug, Ara-M is metabolized by herpes virus thymidine kinase to form 6-methoxypurine arabinonucleoside monophosphate (Ara-MMP) that is a toxic substance.

Alternatively, the drug-dependent suicide gene may be a gene encoding a non-toxic protein that is converted to a toxic substance with the addition of a drug. For example, the drug-dependent suicide gene may encode a protein that does not exhibit toxicity in the monomer form but exhibits toxicity in the multimer (e.g., dimer) form. In this case, the expression "drug that allows a drug-dependent suicide gene to exhibit toxicity" refers to a substance that converts a drug-dependent suicide gene to a toxic substance. For example, when a caspase such as Caspase 9 or Caspase 3 is used as a drug-dependent suicide gene and a dimerizer is used as a drug, Caspase-9 dimer that is a toxic substance having activity is formed as a result of dimerization of the caspase by the dimerizer, thereby enabling apoptosis induction. Examples of such drug-dependent suicide gene that exhibits toxicity in the multimer (e.g., dimer) form include a Fas receptor and FADD.

The expression "sequence capable of allowing a promoter to cause the two genes to be simultaneously expressed" used herein refers to a sequence capable of activating a promoter to translate two types of proteins or peptides. Such sequence may be a sequence having self-cleavage activity, which activates a promoter to allow the promoter to cause two genes to be simultaneously expressed so that two proteins or peptides bound via the sequence are translated and then undergo self-cleavage in the region of the sequence, thereby eventually allowing the two separate proteins or peptides to be translated. Alternatively, the expression "sequence capable of allowing a promoter to cause the two genes to be simultaneously expressed" may be a sequence that attracts a ribosome to initiate the second translation from the middle of mRNA so that two different genes are bicistronically translated from mRNA. One example of the former is the "2A sequence" and one example of the latter is the "IRES (internal ribosome entry site) sequence." More specifically, examples of the 2A sequence include the 2A sequence derived from the foot-and-mouth disease virus and the 2A sequence derived from the equine rhinitis A virus (see Furler, S. et al. (2001), Gene Ther. 8, 864-73; and Hasegawa et al. (2007), Stem Cells 25, 1707-12).

The vector used in the method of the present invention has a recombination cassette containing a promoter region. The term "recombination cassette" refers to a region that allows gene recombination (using a recombinase), which has nucleic acid sequences recognized by a recombinase and a foreign gene insertion site flanked by the nucleic acid sequences. Examples of the recombination cassette include cassettes for *λ* phage recombination such as LR recombination in which recombination takes place between attL at one end of a DNA fragment and attR contained in a donor vector (bacteriophage *λ* integrase or excisionase and an *Escherichia coli* integration host factor protein) or BP recombination in which recombination takes place between attB at one end of a DNA fragment and attP contained in a donor vector (bacteriophage *λ* integrase and an *Escherichia coli* integration host factor protein) (see Landy, A (1989), Ann. Rev. Biochem. 58: 913-949; and Ptashne, M. (1992), A Genetech Switch: Phage (Lambda) And Higher Organisms (Cambridge, MA: Cell Press)) (Gateway (registered trademark) Technology, Invitrogen); Cre-loxP recombination (Cre); FLP-FRT recombination (Flippase); and RS recombination (R Recombinase, GIX Recombinase).

According to the present invention, the term "undifferentiated-cell-specific promoter" refers to a promoter having activity specific to undifferentiated cells and/or tumorigenic cells and causing a marker gene and/or a drug-dependent suicide gene bound downstream thereof to be expressed to a sufficient degree to exhibit the gene function. Examples of such promoter include a telomerase reverse transcriptase (tert) promoter (see Takakura, M. et al., Cancer Res., 59:551-557, 1999), a survivin promoter (see Li, F. et al., (1999) Cancer Res., 59: 3143-3151; LI, F. et al., (1999) Biochem. J., 344: 305-311), an Aurora kinase promoter, a HIF (hypoxia-inducible factor)-responsive element (HRE) promoter, a Grp78 promoter, an L-plastin promoter, a hexokinase II promoter, an Oct3/4 promoter, an Nanog promoter, an SOX2 promoter, a CRIPTO promoter, a DAX1 promoter, an ERas promoter, an Fgf4 promoter, an Esg1 promoter, a Rex1 promoter, a Zfp296 promoter, a UTF1 promoter, a GDF3 promoter, a Sall4 promoter, a Tbx3 promoter, a Tcf3 promoter, a DNMT3L promoter, a DNMT3B promoter, and a miR-290 promoter.

The expression "operably linked to" used herein means a state in which expression of the nucleic acid sequence of a gene of interest is allowed and the nucleic acid sequence is linked to a regulatory sequence (promoter sequence) (in an *in vitro* transcription/translation system or, if a vector is introduced into host cells, in host cells).

In the invention, the viral vector isa lentiviral vector.

### EFFECTS

The viral vector and methods of the disclosure can impart both functions of labeling and cell killing at one instance of gene transfer because a marker gene and a toxic gene are bound via a sequence capable of allowing a promoter to cause the two genes to be simultaneously expressed. In addition, since an arbitrary promoter can be conveniently incorporated into the vector using a recombination system, it is possible to efficiently and rapidly prepare a variety of viral vectors by selecting a plurality of candidate promoters appropriate for desired cells that are induced to differentiate or desired timing of cell removal (before transplantation/after transplantation), for example. Further, the vector of the present disclosure can be used for conveniently analyzing whether or not a promoter has activity specific to undifferentiated cells or tumorigenic cells. Moreover, if a drug-dependent suicide gene is positioned under the control of the promoter sequence specific to undifferentiated cells or tumorigenic cells in the vector of the present disclosure, the vector is configured not to exert the cell killing function without drug-dependent induction. Even if the vector is introduced into all stem cells before differentiation induction treatment, cells do not die unless a drug is added. Therefore, it is possible to perform gene transfer before differentiation induction so as to use only stem cells transfected with a gene for differentiation induction, thereby preventing a failure in gene transfer. Moreover, since a drug-dependent suicide gene carried by the vector of the present disclosure is of a human-derived enzyme, immunogenicity issues can be resolved. That is, by introducing the viral vector containing an undifferentiated-cell-specific promoter of the present disclosure into all stem cells before differentiation treatment, it is possible to specifically and comprehensively label/kill remaining undifferentiated cells after differentiation treatment. Accordingly, the issue of tumorigenesis of remaining undifferentiated cells in regenerative medicine can be resolved.

### BRIEF DESCRIPTION OF THE DRAWINGS

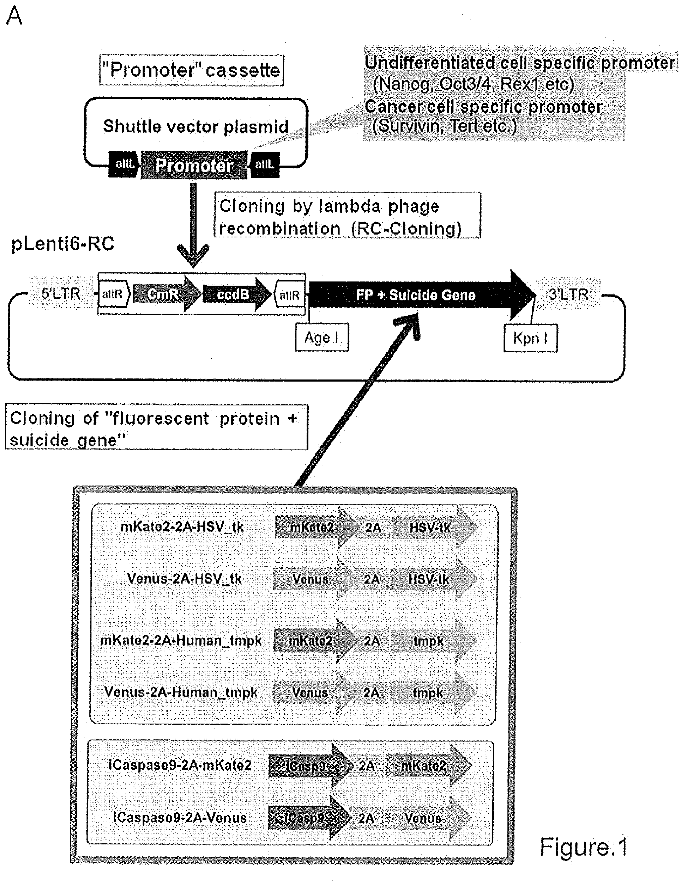

Fig. 1 schematically shows a vector construct. A "fluorescent protein + suicide gene" cassette prepared by cloning using a restriction enzyme and In-Fusion (registered trademark) cloning was introduced into a lentivirus vector plasmid (pLentI6-RC) containing a recombination cassette (RC), thereby producing "pLenti6-RC-fluorescent protein-2A-suicide gene." For recombination of the promoter region, a promoter subcloned into a shuttle vector plasmid by Gateway (registered trademark) cloning ("Promoter" cassette) was inserted into the RC portion of pLenti6-RC-fluorescent protein-2A-suicide gene, thereby completing a plasmid: "pLenti6-Promoter- fluorescent protein -2A-suicide gene."

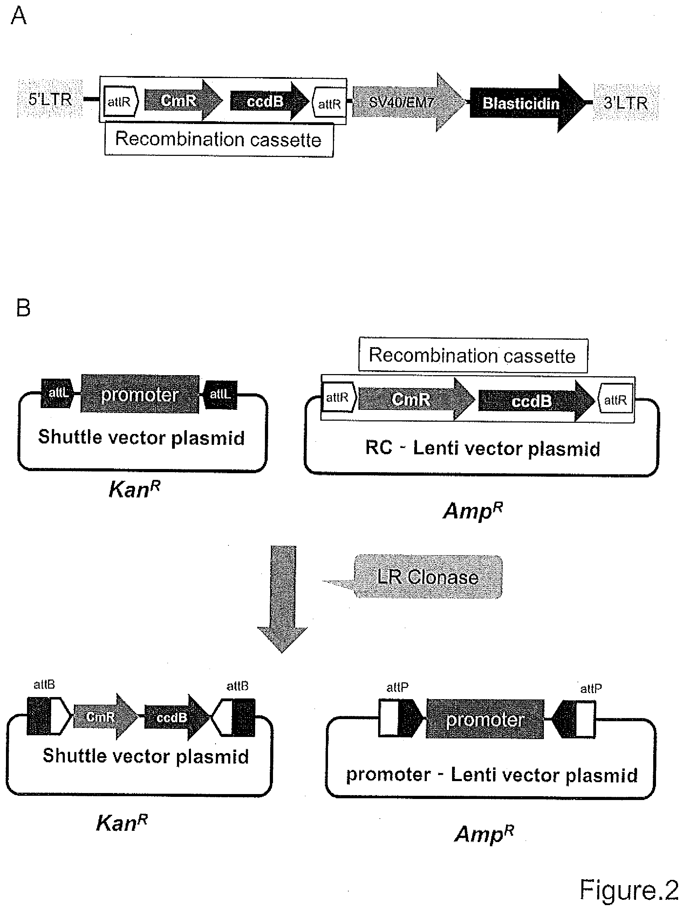

Fig. 2 schematically shows the configuration of pLenti6-RC and promoter insertion. A configuration of a lentivirus vector plasmid (pLenti6-RC) containing RC is shown in A. Insertion of a promoter cassette using LR Clonase is shown in B. pLenti6-RC and a shuttle vector plasmid for gene recombination contain homologous att sequences. A shuttle vector plasmid into which a promoter of interest has been cloned, pLenti6-RC, and LR clonase serving as a recombinase are allowed to react so that the promoter sequence is incorporated into the pLenti vector.

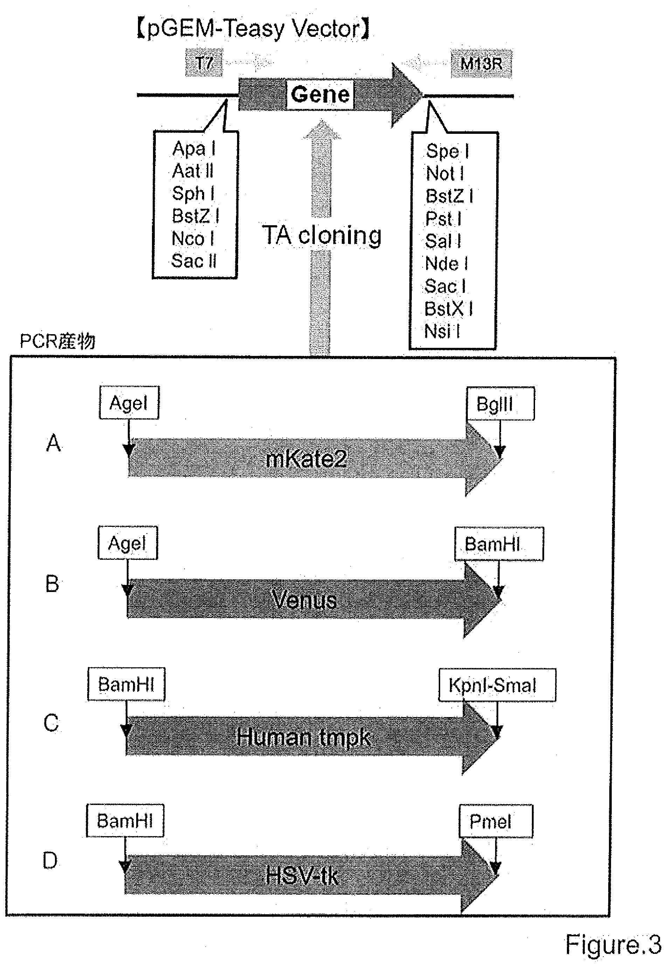

Fig. 3 schematically shows restriction enzyme addition by PCR and cloning into a pGEM-Teasy vector. Subcloning of fluorescent protein genes (mKate2: A, Venus: B) and suicide genes (human-tmpk: C, HSV-tk: D) used for vector construction was performed, followed by TA cloning into a pGEM-Teasy Cloning vector. A restriction enzyme sequence was added to each gene as shown in the figure.

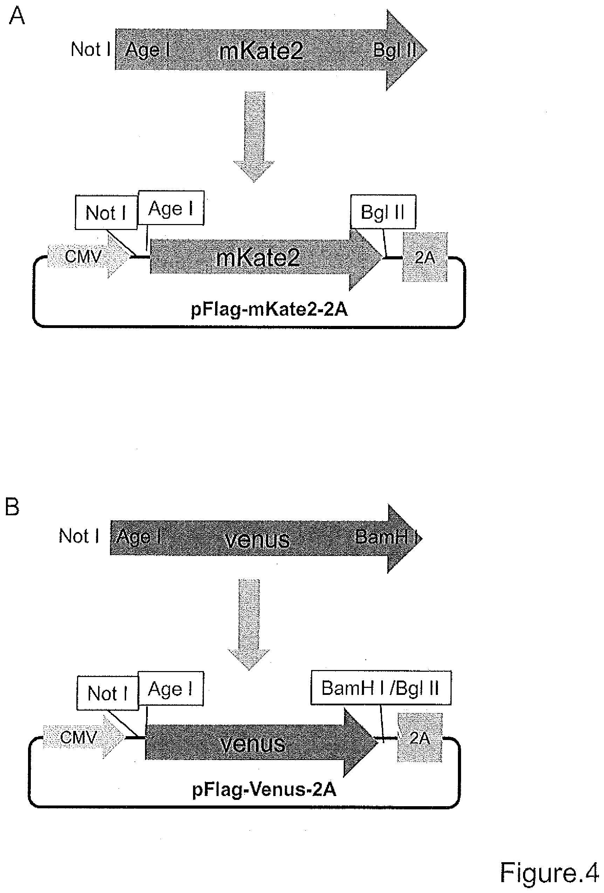

Fig. 4 schematically shows cloning of a fluorescent protein gene into a pFlag-CMV-2A vector. mKate2 (A) and Venus (B) subcloned into pGEM-Teasy were spliced out using a restriction enzyme and inserted between CMV and 2A of pFlag-CMV-2A, which had been cleaved using NotI/BglII.

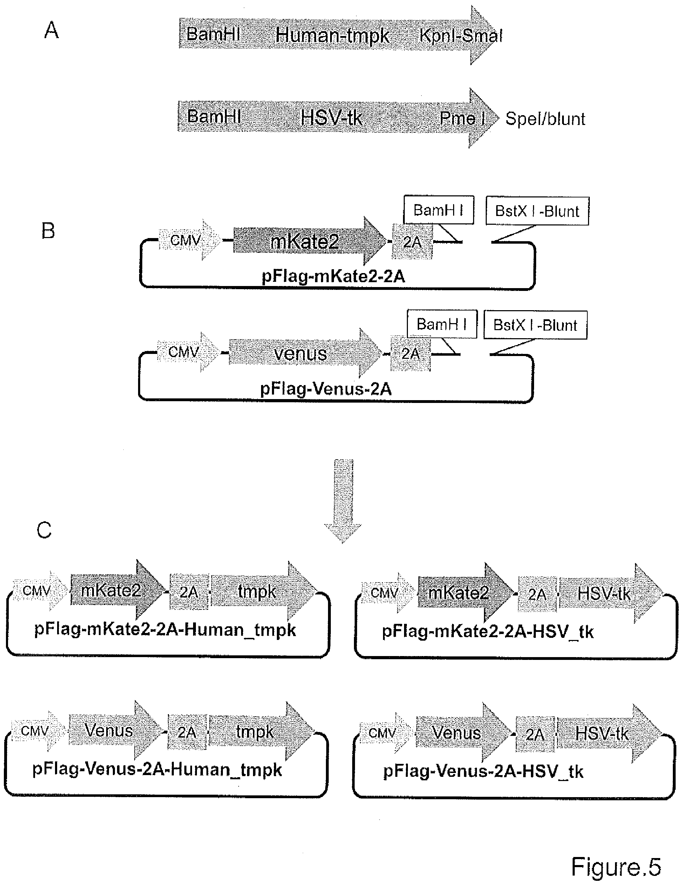

Fig. 5 schematically shows preparation of a "fluorescent protein + suicide gene" expression cassette. Human-tmpk and HSV-tk subcloned into pGEM-Teasy were spliced out using a restriction enzyme (A) and inserted into pFlag-CMV-fluorescent protein-2A which had been cleaved with BamHI and BstXI (blunting) (B). Fig. 5C schematically shows completed plasmids.

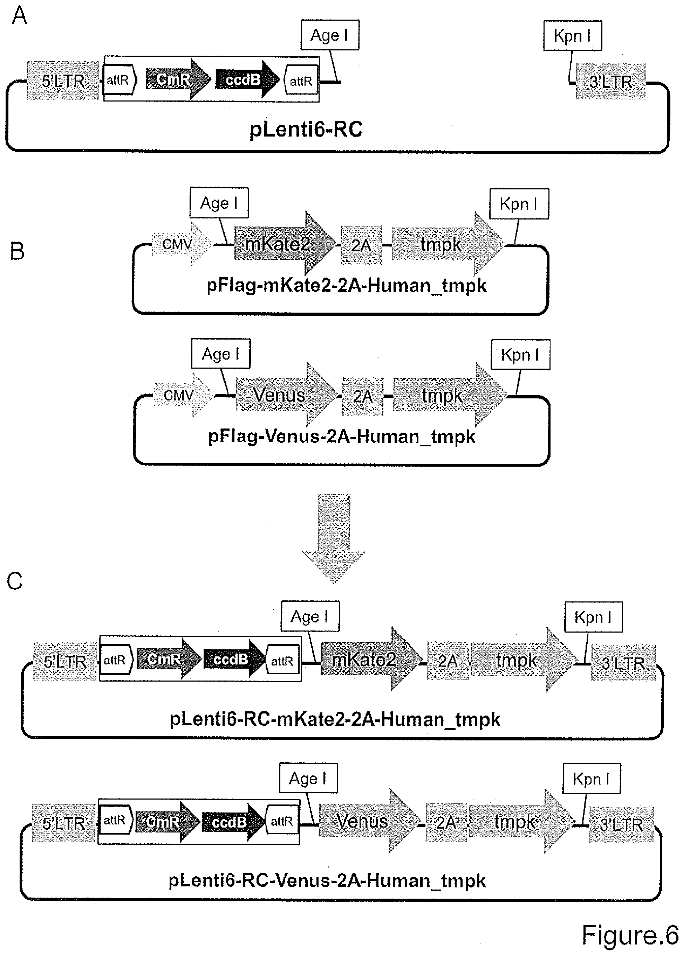

Fig. 6 schematically shows insertion of a "fluorescent protein + suicide gene" expression cassette into a pLenti6-RC plasmid. The two different "fluorescent protein + suicide gene" expression cassettes (B) shown in Fig. 5 were respectively inserted between the Agel and KpnI sites of pLenti6-RC (A) containing RC shown in Fig. 2 (C).

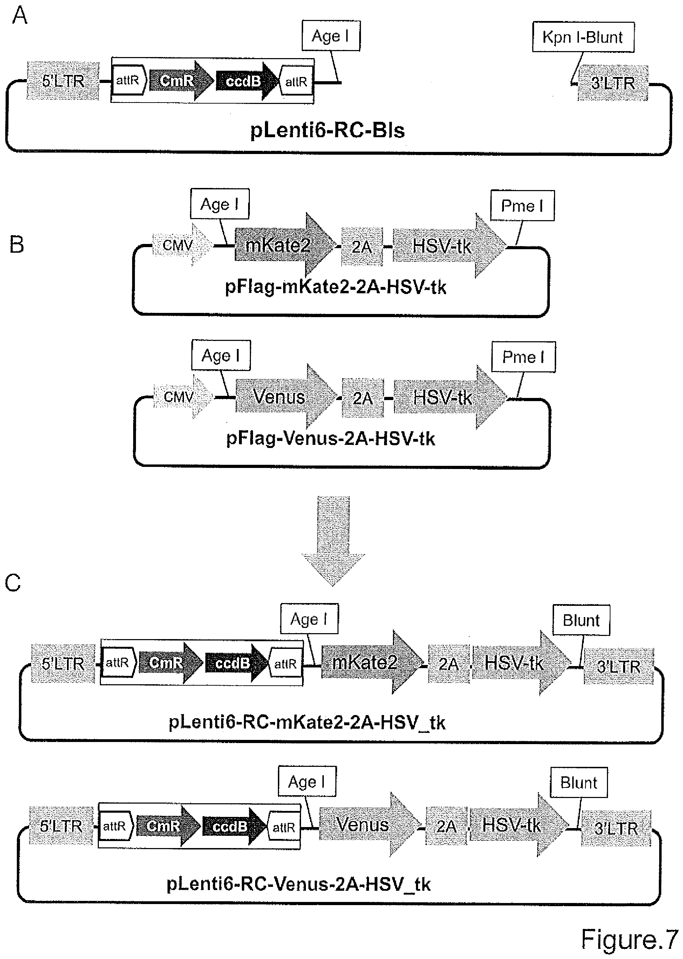

Fig. 7 schematically shows insertion of a "fluorescent protein + suicide gene" expression cassette into a pLenti6-RC plasmid. The two different "fluorescent protein + suicide gene" expression cassettes (B) shown in Fig. 5 were respectively inserted between the Agel and KpnI sites of pLenti6-RC (A) containing RC shown in Fig. 2 (C).

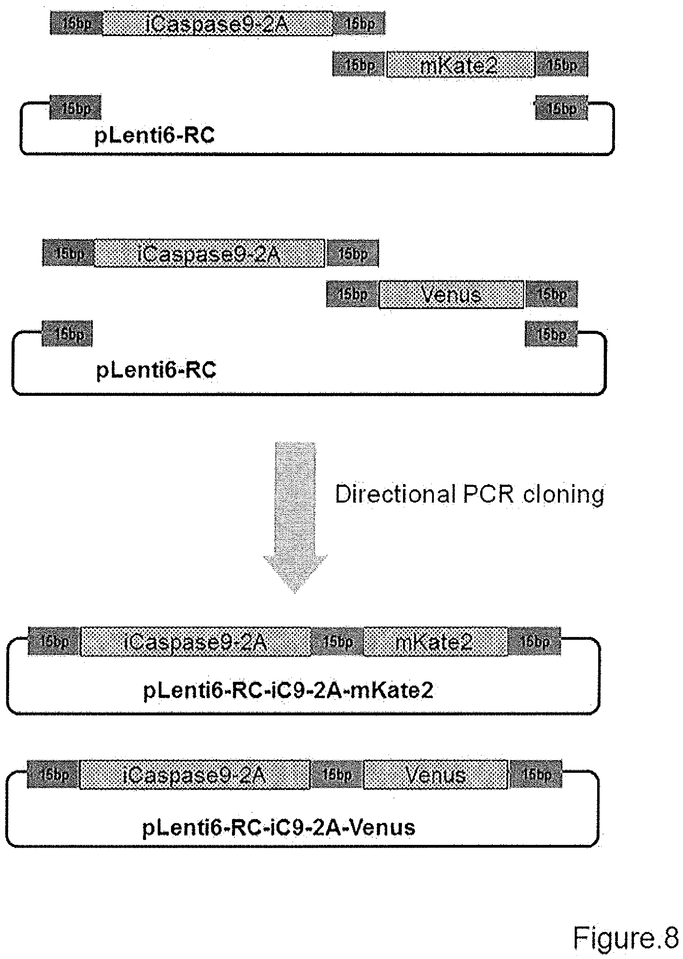

Fig. 8 schematically shows preparation of pLenti6-RC containing an "iCaspase + fluorescent protein" expression cassette. pLenti6-RC was used as a vector and a primer was designed to have 15-bp homologous sequences at both ends so that iCaspase9-2A and a fluorescent protein gene were inserted downstream of RC, followed by PCR. These were mixed and allowed to react using the IN-FUSION enzyme for cloning.

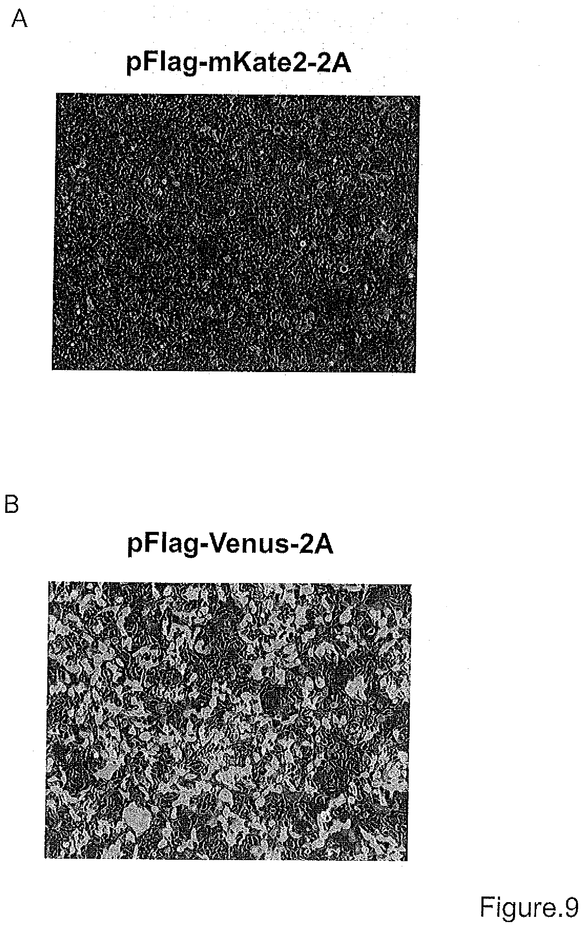

Fig. 9 shows images confirming the expression of subcloned fluorescent proteins. The expression of mKate2 (A) and Venus (B) inserted into pFlag-CMV-2A was confirmed. Each image shows the expression of the prepared plasmid introduced into HEK293 cells, which was confirmed 48 hours later using a fluorescence microscope.

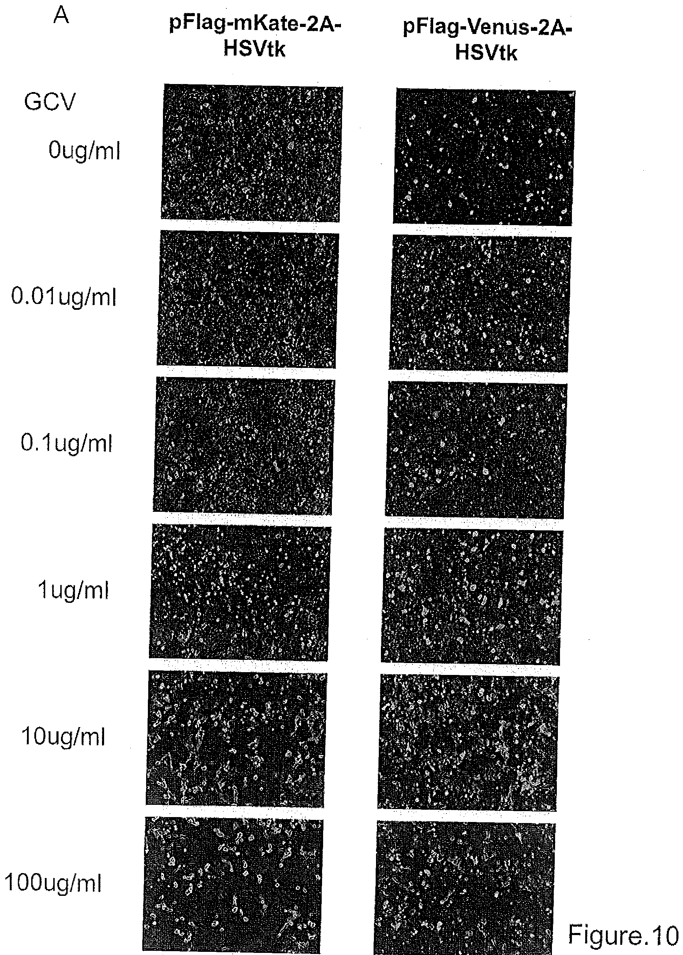

Fig. 10A shows images confirming the expression of a "fluorescent protein + suicide gene." The plasmid shown in Fig. 2B was introduced into HEK293 cells, and GCV was administered at different concentrations of 0, 0.01, 0.1, 1, 10, and 100 µg/mL starting from 48 hours later for 3 days. Cells transfected with the plasmid containing HSV-tk were observed using a fluorescence microscope, and the fluorescence expression of mKate2 (red) and Venus (green) was confirmed after GCV administration in a time-dependent manner.

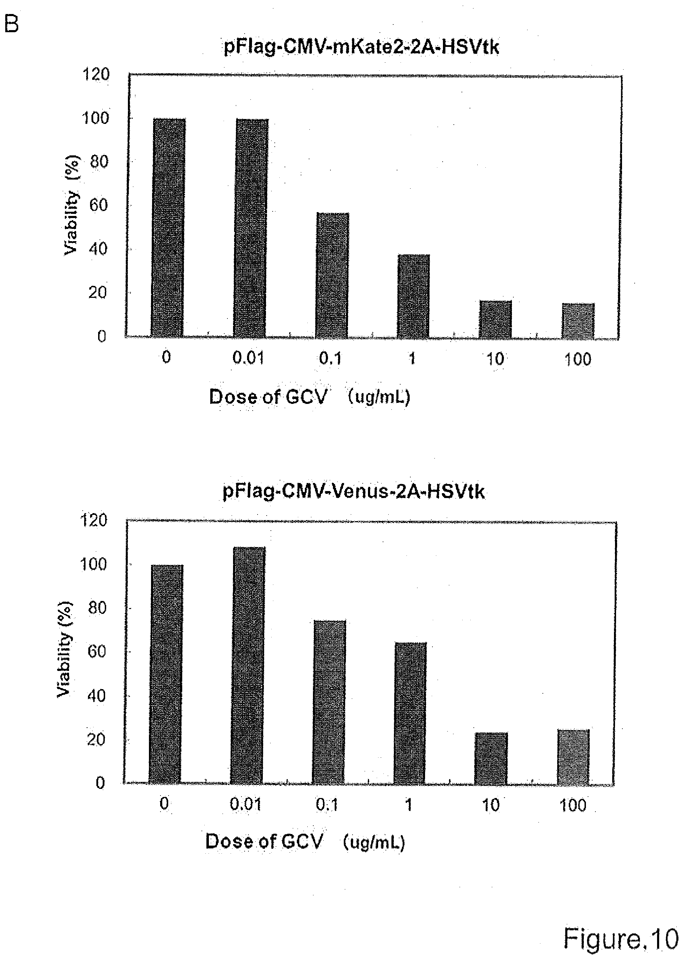

Fig. 10B shows the results of counting the number of viable cells after GCV administration for the cells shown in Fig. 10A. Figures on the vertical axis are expressed by percentage (%) with respect to the number of viable cells at a drug concentration of 0 µg/mL (100%).

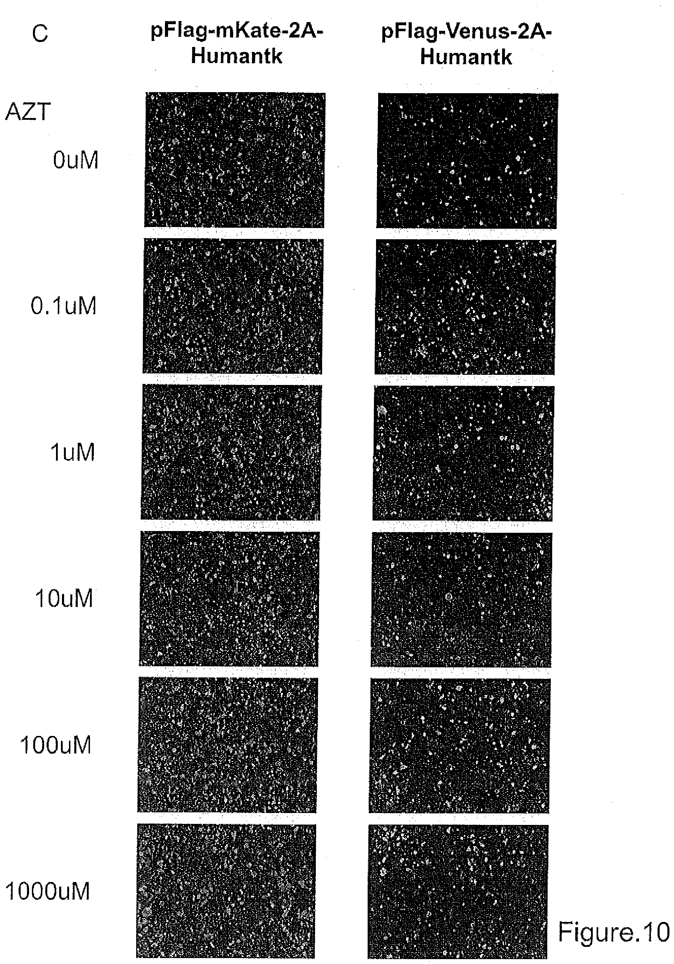

Fig. 10C shows images confirming the expression of a "fluorescent protein + suicide gene" cassette. The plasmid shown in Fig. 2B was introduced into HEK293 cells, and AZT was administered at different concentrations of 0, 0.1, 1, 10, 100, 1000 µM starting from 48 hours later for 3 days. Cells transfected with the plasmid containing human-tmpk were observed using a fluorescence microscope, and the fluorescence expression of mKate2 (red) and Venus (green) was confirmed after AZT administration in a time-dependent manner.

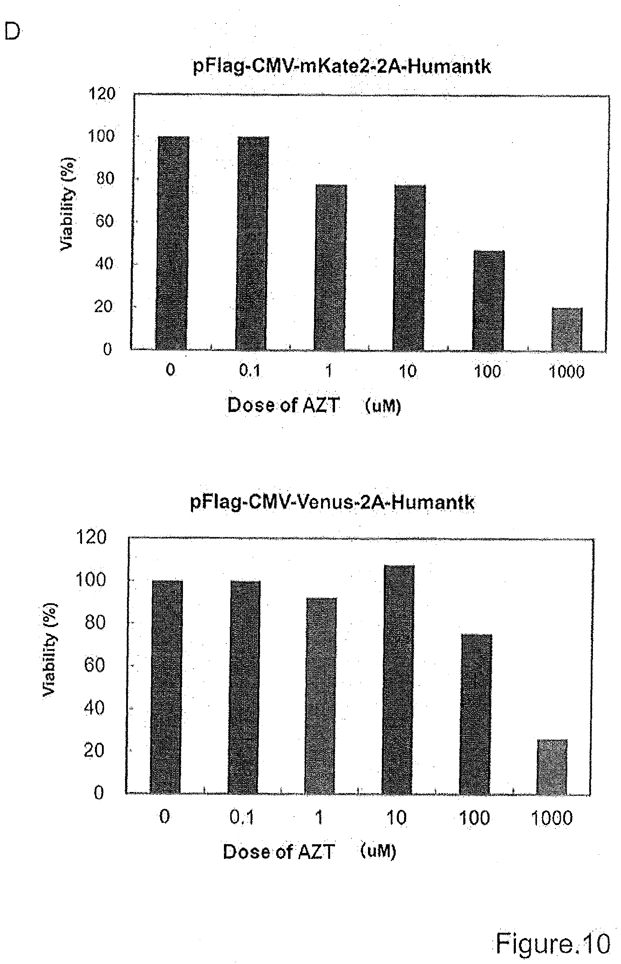

Fig. 10D shows the results of counting the number of viable cells after AZT administration for the cells shown in Fig. 10C. Figures on the vertical axis are expressed by percentage (%) with respect to the number of viable cells at a drug concentration of 0 µg/mL (100%).

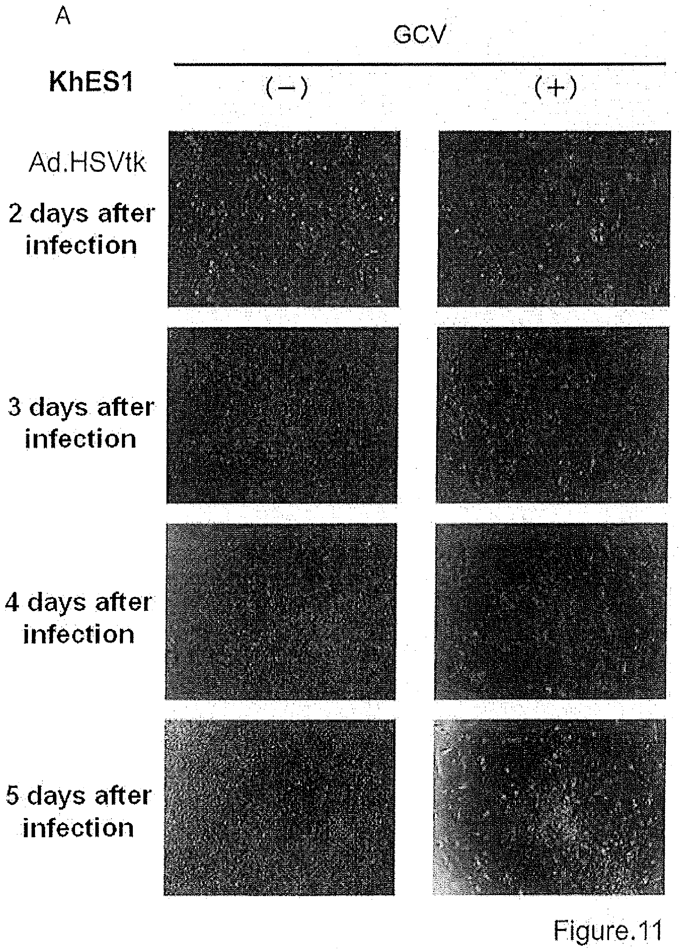

Fig. 11A shows images indicating GCV sensitivity in KhES1 cells transfected with HSV-tk. The difference in cytotoxic effects due to the administration or non-administration of GCV into KhES1 cells infected with Ad.CAG-HSV-tk at MOI = 10 was observed in a time-dependent manner. Images on the left side show the results for the no-GCV-treatment group, and images on the right side show the results for the GCV-treatment group.

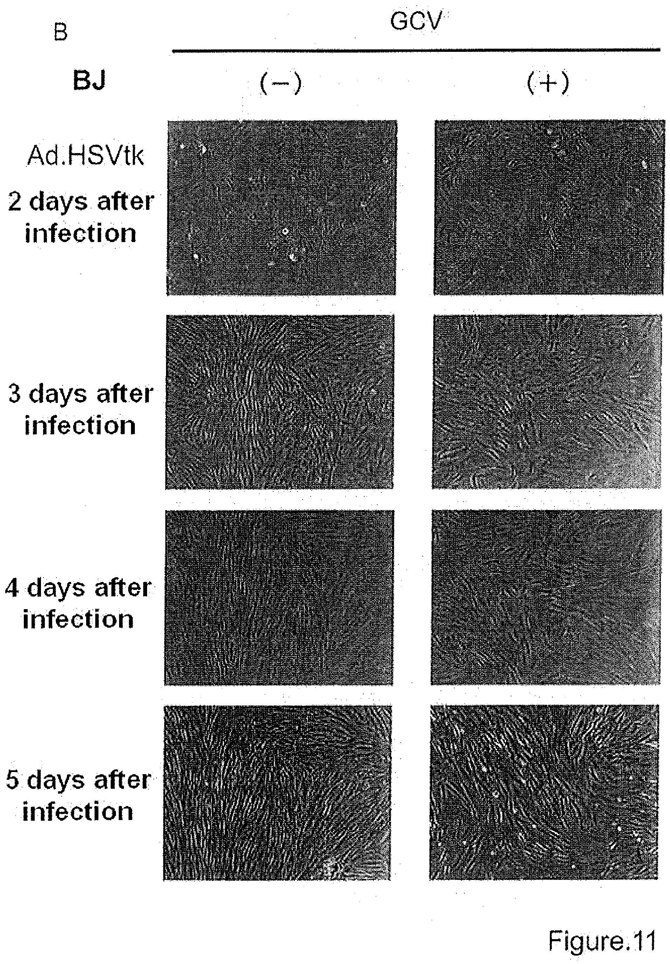

Fig. 11B shows images indicating GCV sensitivity in BJ cells transfected with HSV-tk. The difference in cytotoxic effects due to the administration or non-administration of GCV into BJ cells infected with Ad.CAG-HSV-tk at MOI = 10 was observed in a time-dependent manner. Images on the left side show the results for the no-GCV-treatment group, and images on the right side show the results for the GCV-treatment group.

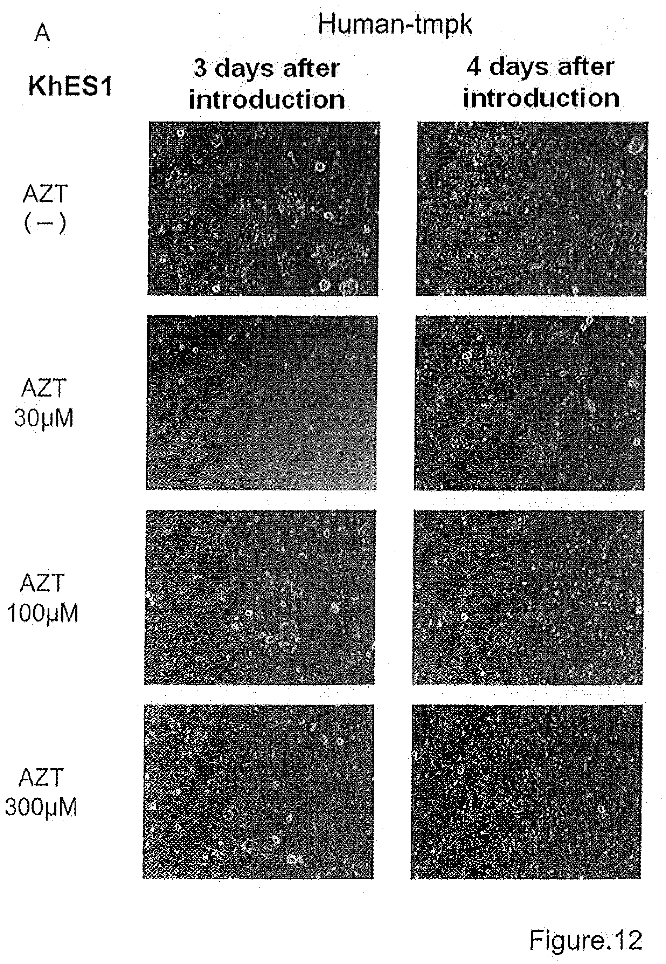

Fig. 12A shows images indicating AZT sensitivity in KhES1 cells transfected with human-tmpk. KhES1 cells were transfected with pHR'-cPPT-EF-Tmpk (F105Y), AZT was administered at 0, 30, 100, and 300 µM starting from 24 hours later, and cytotoxic effects were confirmed 3 days and 4 days later.

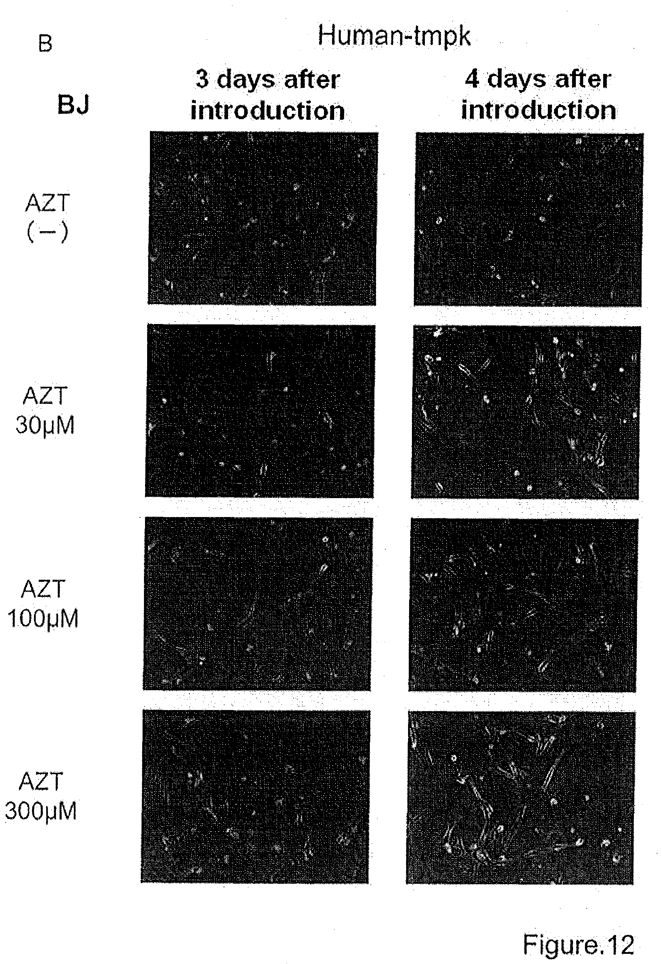

Fig. 12B shows images indicating AZT sensitivity in BJ cells transfected with human-tmpk. BJ cells were transfected with pHR'-cPPT-EF-Tmpk (F105Y), AZT was administered at 0, 30, 100, and 300 µM starting from 24 hours later, and cytotoxic effects were confirmed 3 days and 4 days later.

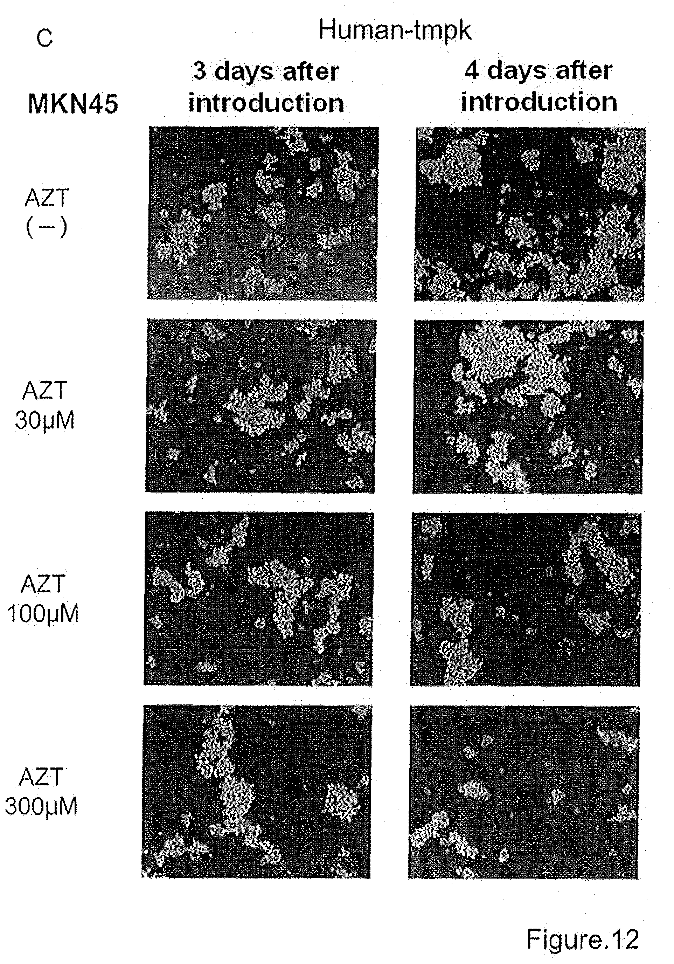

Fig. 12C shows images indicating AZT sensitivity in MKN45 cells transfected with human-tmpk. MKN45 cells were transfected with pHR'-cPPT-EF-Tmpk (F105Y), AZT was administered at 0, 30, 100, and 300 µM starting from 24 hours later, and cytotoxic effects were confirmed 3 days and 4 days later.

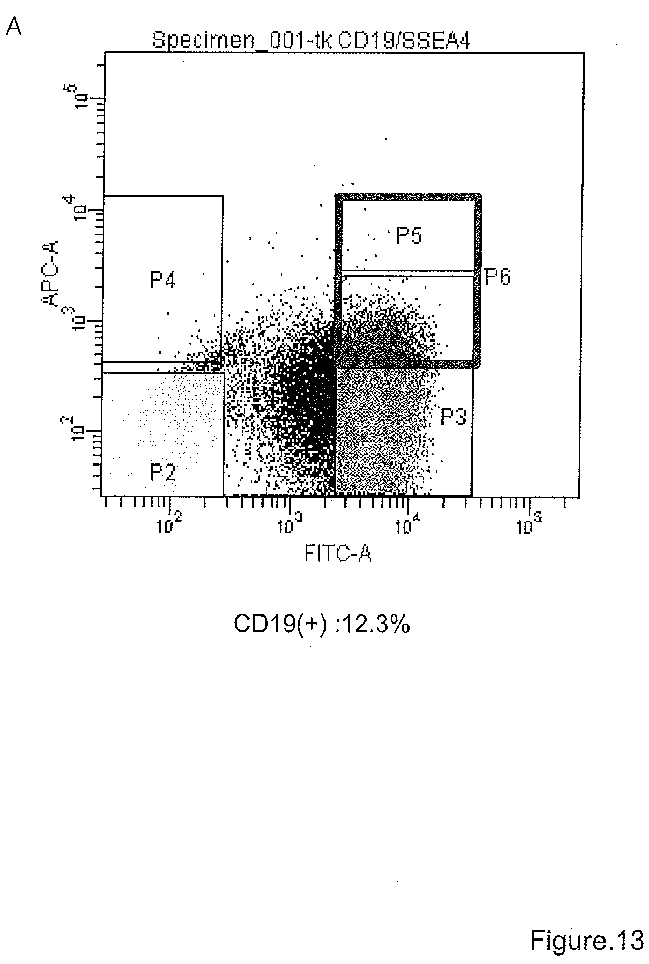

Fig. 13A shows a graph indicating the results of confirming sorted cells constitutively expressing Human tmpk (CD19-positive cells). KhES1 cells (1 × 10⁶ cells) were labeled with two different substances, which were Alexa Fluor 488 anti-Human SSEA4 and anti-Human CD19-APC, and the proportion of SSEA4/CD19-positive cells was analyzed using a flow cytometer.

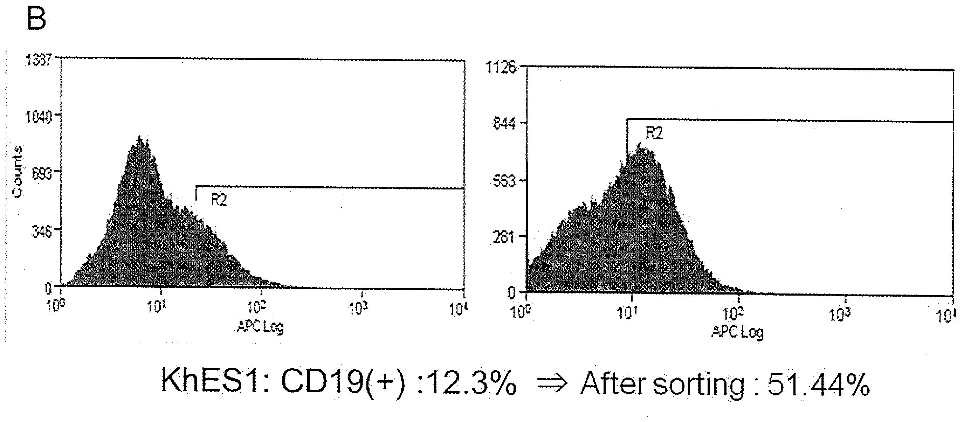

Fig. 13B shows graphs indicating the results of confirming sorted cells constitutively expressing Human tmpk (CD19-positive cells). KhES1 cells (1 × 10⁶ cells) were labeled with anti-Human CD19-APC, and the proportion of CD19-positive cells was analyzed using a flow cytometer. The left graph shows analysis results before sorting and the right graph shows analysis results after sorting.

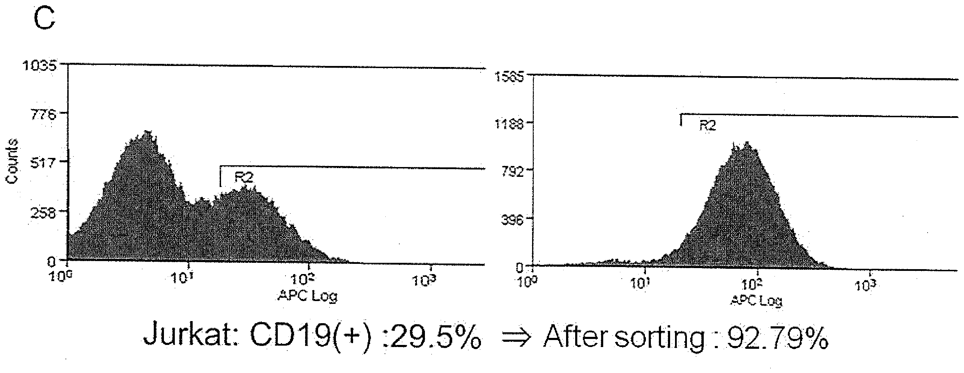

Fig. 13C shows graphs indicating the results of confirming sorted cells constitutively expressing Human tmpk (CD19-positive cells). Jurkat cells (1 × 10⁶ cells) were labeled with anti-Human CD19-APC, and the proportion of CD19-positive cells was analyzed using a flow cytometer. The left graph shows analysis results before sorting and the right graph shows analysis results after sorting.

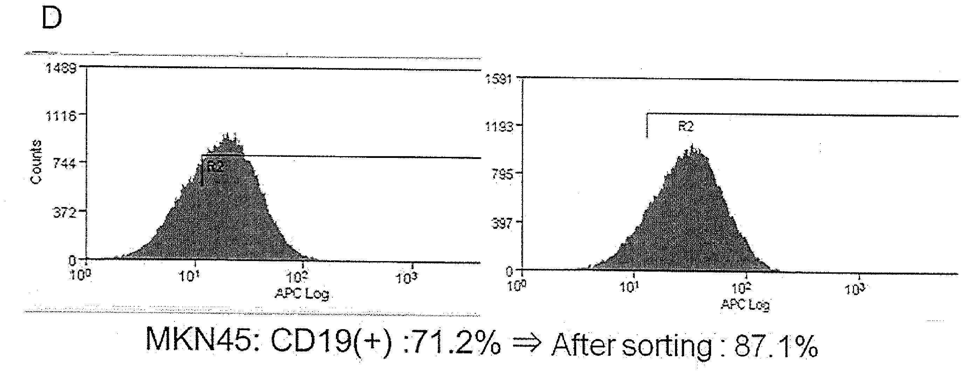

Fig. 13D shows graphs indicating the results of confirming sorted cells constitutively expressing Human tmpk (CD19-positive cells). MKN45 cells (1 × 10⁶ cells) were labeled with anti-Human CD19-APC, and the proportion of CD19-positive cells was analyzed using a flow cytometer. The left graph shows analysis results before sorting and the right graph shows analysis results after sorting.

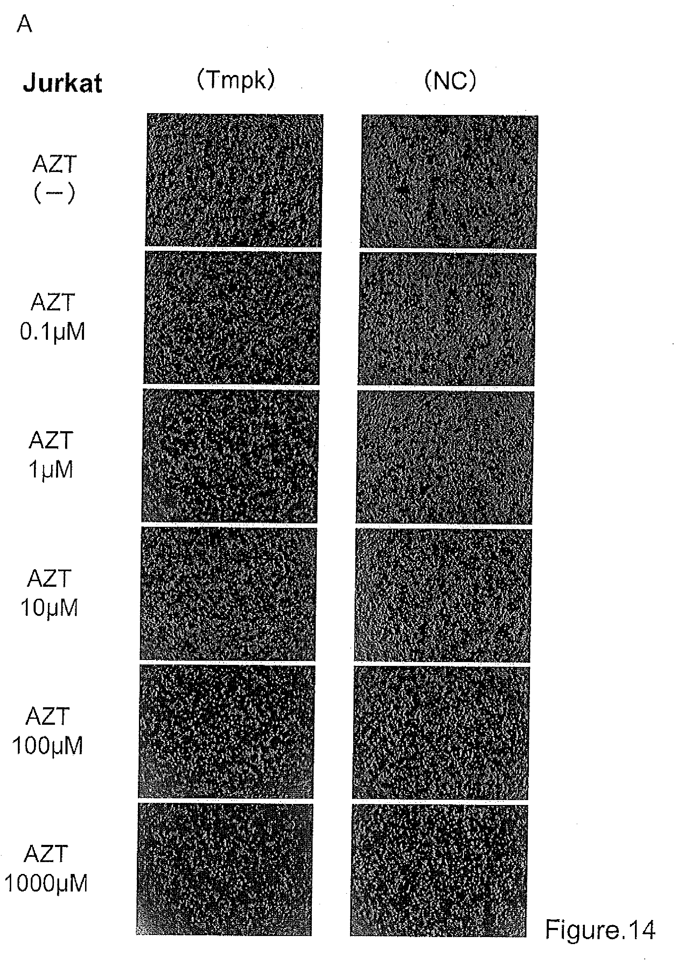

Fig. 14A shows images confirming the results of drug sensitivity in Jurkat cells constitutively expressing Human tmpk. Sorted human-cells constitutively expressing tmpk (tmpk) and non-infected cells (negative control: NC) were seeded on a 24-well plate at 1 × 10⁵ cells per well. AZT was administered at 0, 0.1, 1, 10, 100, and 1000 µM to both tmpk and NC groups starting from the following day for 3 days. Each image shows the appearance of Jurkat cells 3 days after AZT treatment. The images on the left side show the group of cells constitutively expressing tmpk and the images on the right side show the NC group.

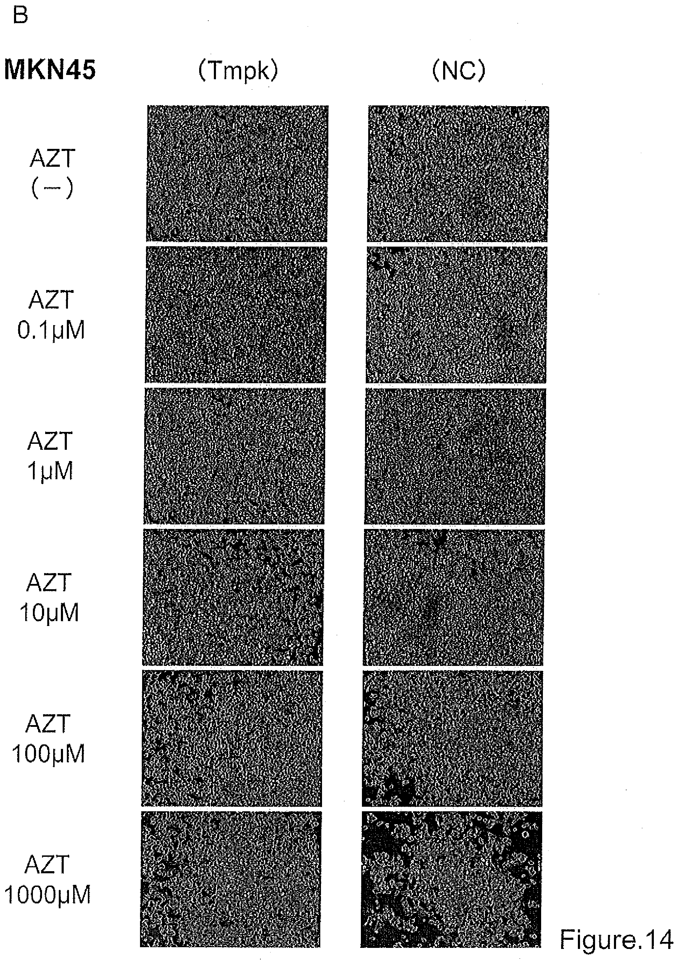

Fig. 14B shows images confirming the results of drug sensitivity in MKN cells constitutively expressing Human tmpk. Sorted human-cells constitutively expressing tmpk (tmpk) and non-infected cells (negative control: NC) were seeded on a 24-well plate at 1 × 10⁵ cells per well. AZT was administered at 0, 0.1, 1, 10, 100, and 1000 µM to both tmpk and NC groups starting from the following day for 3 days. Each image shows the appearance of MKN cells 3 days after AZT treatment. The images on the left side show the group of cells constitutively expressing tmpk and the images on the right side show the NC group.

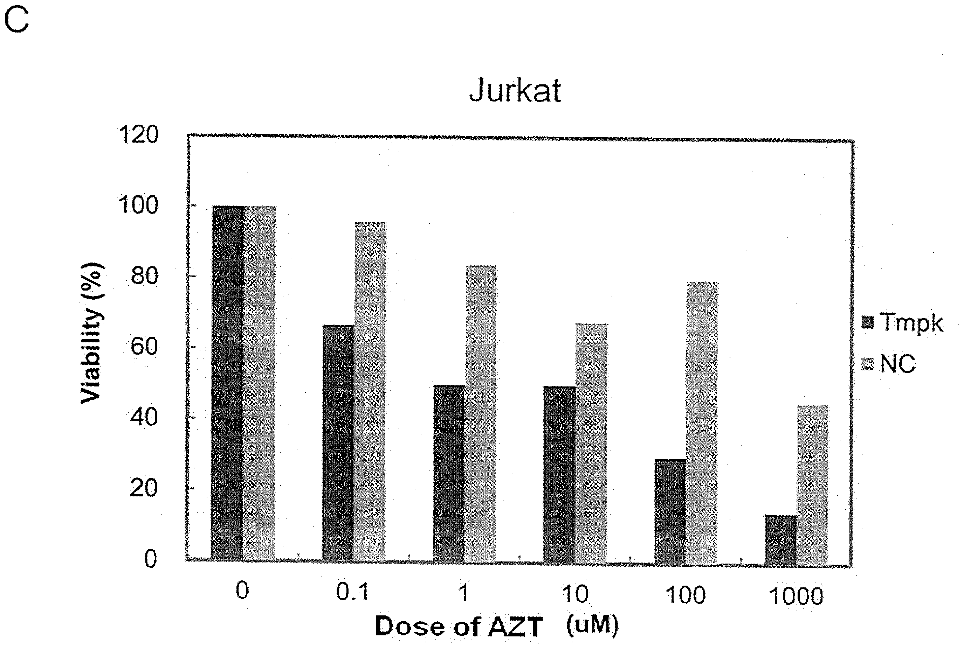

Fig. 14C shows a graph indicating the results of determining the number of viable Jurkat cells by cell count 3 days after administration in the experiment of Fig. 14A. Figures on the vertical axis are expressed by percentage (%) with respect to the number of viable cells at a drug concentration of 0 µg/mL (100%).

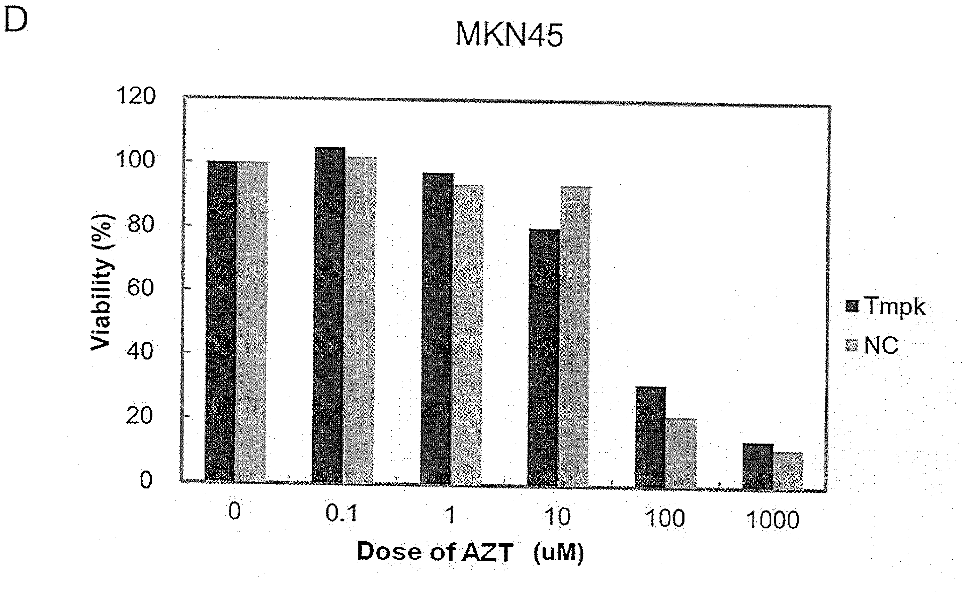

Fig. 14D shows a graph indicating the results of determining the number of viable MKN45 cells by cell count 3 days after administration in the experiment of Fig. 14A. Figures on the vertical axis are expressed by percentage (%) with respect to the number of viable cells at a drug concentration of 0 µg/mL (100%).

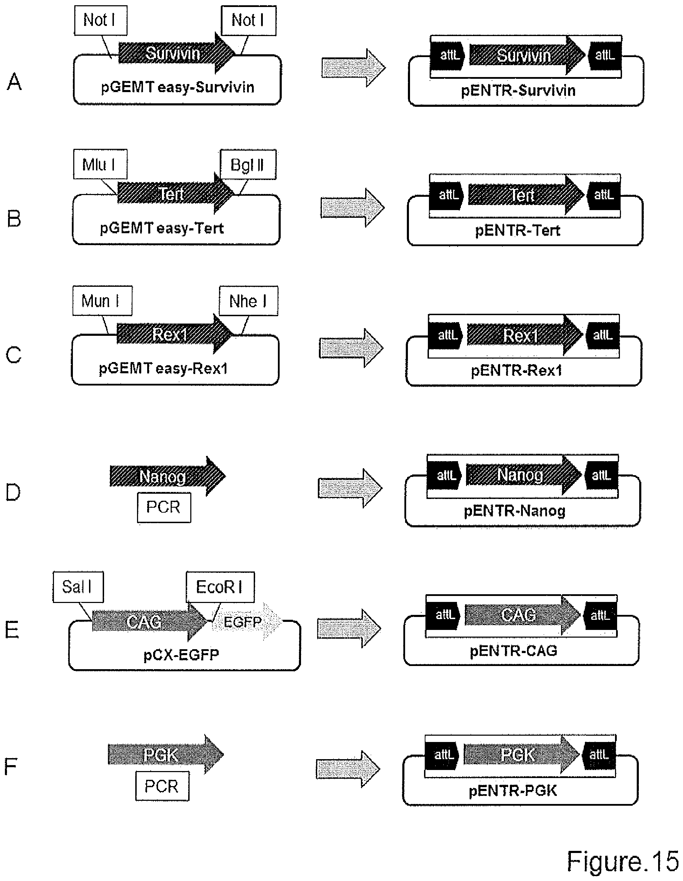

Fig. 15 shows the following conditions: A: the survivin promoter region serving as an undifferentiated-cell-specific promoter candidate was spliced out using NotI from the preliminarily prepared pGEMT-easy-survivin, subjected to blunting treatment, and subcloned into pENTR-vector, thereby constructing pENTR-survivin; B: the tert promoter region serving as an undifferentiated-cell-specific promoter candidate was spliced out using MluI/BglII from the preliminarily prepared pGEMT-EASY-tert, subjected to blunting treatment, and subcloned into pENTR-vector, thereby constructing pENTR-Tert; C: the Rex1 promoter region serving as an undifferentiated-cell-specific promoter candidate was spliced out using MunI/NheI from the preliminarily prepared pGEMT-easy-Rex1, subjected to blunting treatment, and subcloned into pENTR-vector, thereby constructing pENTR-Rex1; D: an extract of genome DNA from mice ES cells (mES) was used as a material for an Nanog promoter serving as an undifferentiated-cell-specific promoter candidate, a primer having a 5' CACC overhang for pENTR-vector cloning of the Nanog promoter region and a 3' end primer were prepared for amplification by PCR, and the amplification product was subcloned into pENTR-vector, thereby constructing pENTR-Nanog; E: the CAG promoter region serving as an undifferentiated-cell-specific promoter candidate was spliced out using SalI/EcoRI from the preliminarily prepared pCX-EGFP, subjected to blunting treatment, and subcloned into pENTR-vector, thereby constructing pENTR-CAG; and F: a PGK promoter serving as a constitutive promoter candidate was amplified by PCR using a primer having a 5' CACC overhang for pENTR-vector cloning and a primer having the 3' PstI site, and the amplification product was subcloned into pENTR-vector, thereby constructing pENTR-PGK.

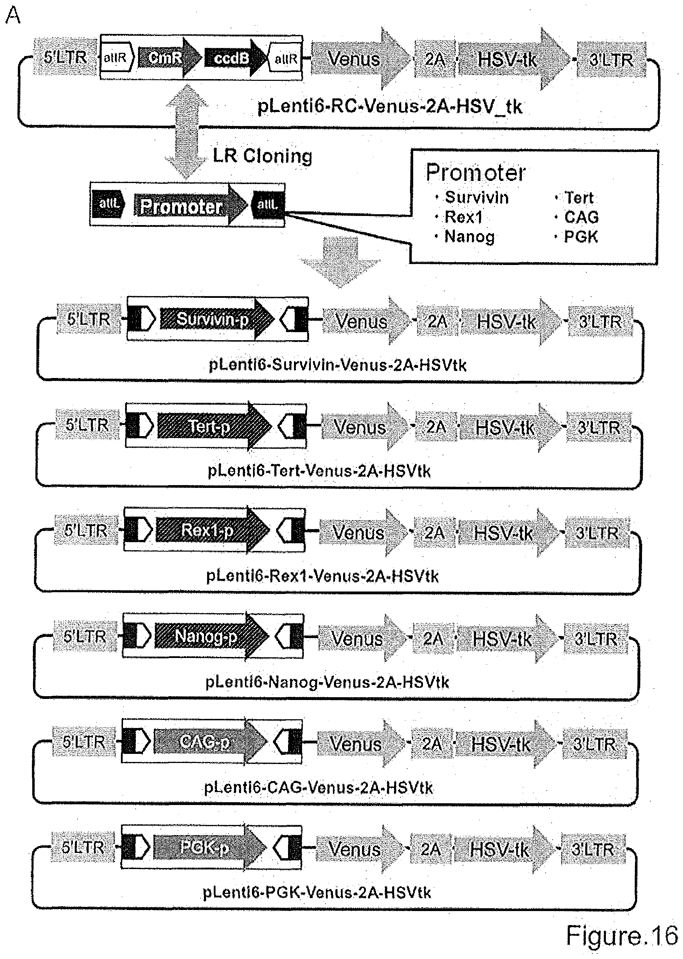

Fig. 16A shows insertion of various promoters into the RC portion of pLenti6-RC-Venus-2A-HSV-tk of the plasmids shown in Fig. 7 via recombination. Survivin, tert, Rex1, and Nanog were inserted as undifferentiated-cell-specific promoters, and constitutive promoters (CAG and PGK) were inserted as controls.

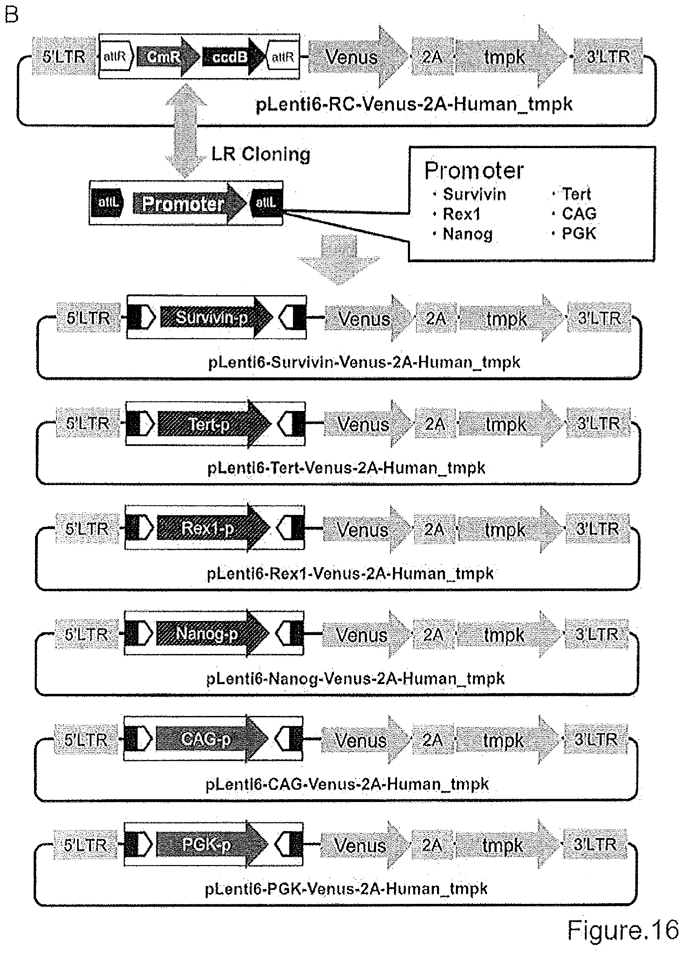

Fig. 16B shows insertion of various promoters into the RC portion of pLenti6-RC-Venus-2A-human-tmpk of the plasmids shown in Fig. 7 via recombination. Survivin, tert, Rex1, and Nanog were inserted as undifferentiated-cell-specific promoters, and constitutive promoters (CAG and PGK) were inserted as controls.

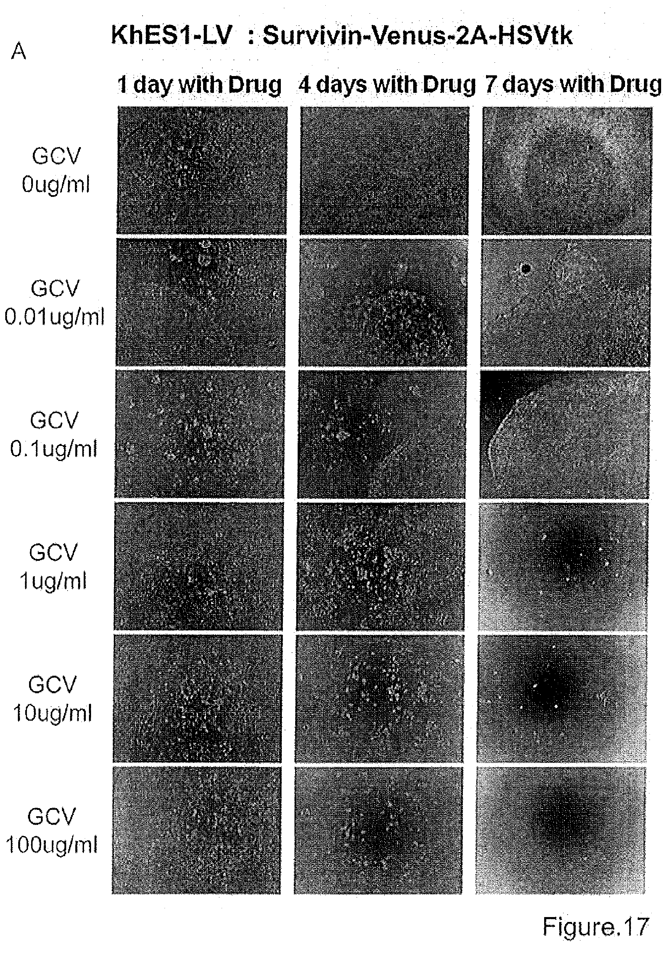

Fig. 17A shows images confirming the expression of Venus in KhES1 cells infected with the lentiviral vector having survivin-Venus-2A-HSV-tk. The images show the appearance of the cells on Days 1, 4, and 7 after GCV treatment from the left.

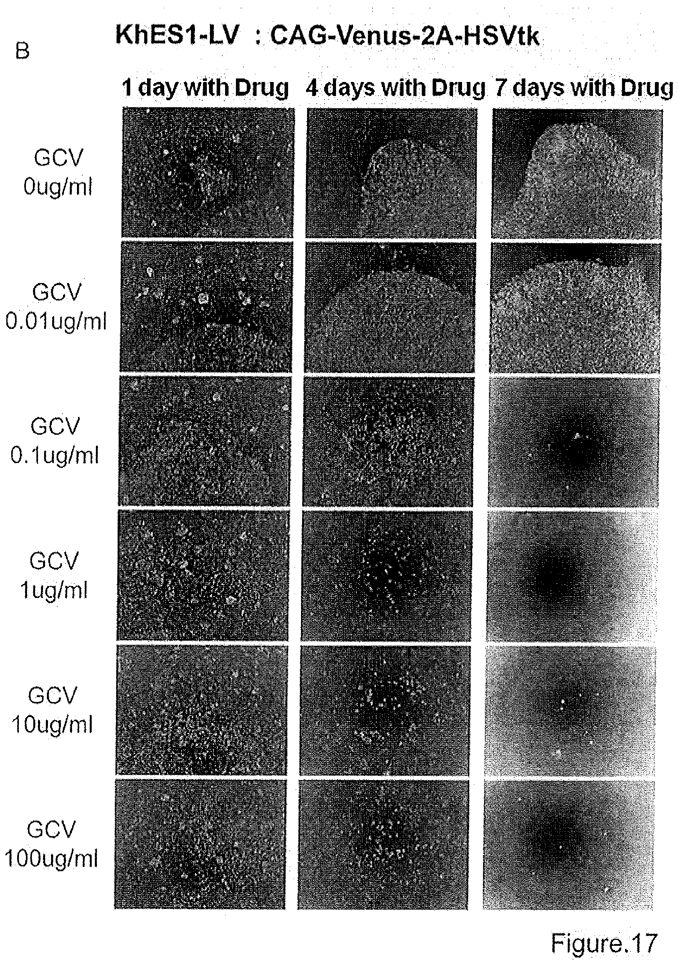

Fig. 17B shows images confirming the expression of Venus in KhES1 cells infected with the lentiviral vector having CAG-Venus-2A-HSV-tk. The images show the appearance of the cells on Days 1, 4, and 7 after GCV treatment from the left.

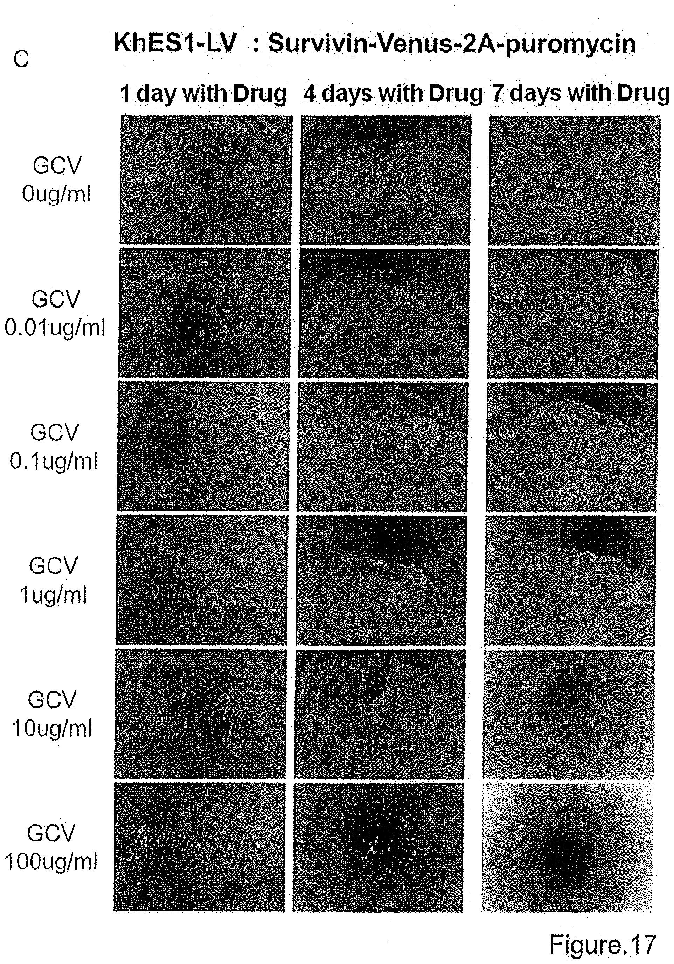

Fig. 17C shows images confirming the expression of Venus in KhES1 cells infected with the lentiviral vector having survivin-Venus-2A-puromycin lacking HSV-tk. The images show the appearance of the cells on Days 1, 4, and 7 after GCV treatment from the left.

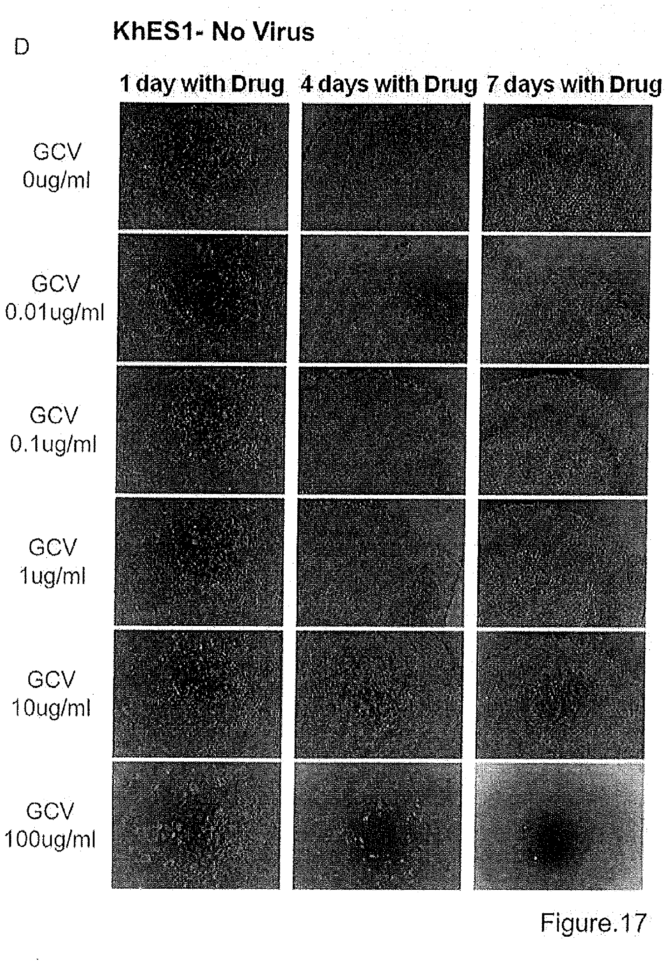

Fig. 17D shows images of cells infected with KhES1 cells that had not been infected with a lentiviral vector (No virus). The images show the appearance of the cells on Days 1, 4, and 7 after GCV treatment from the left.

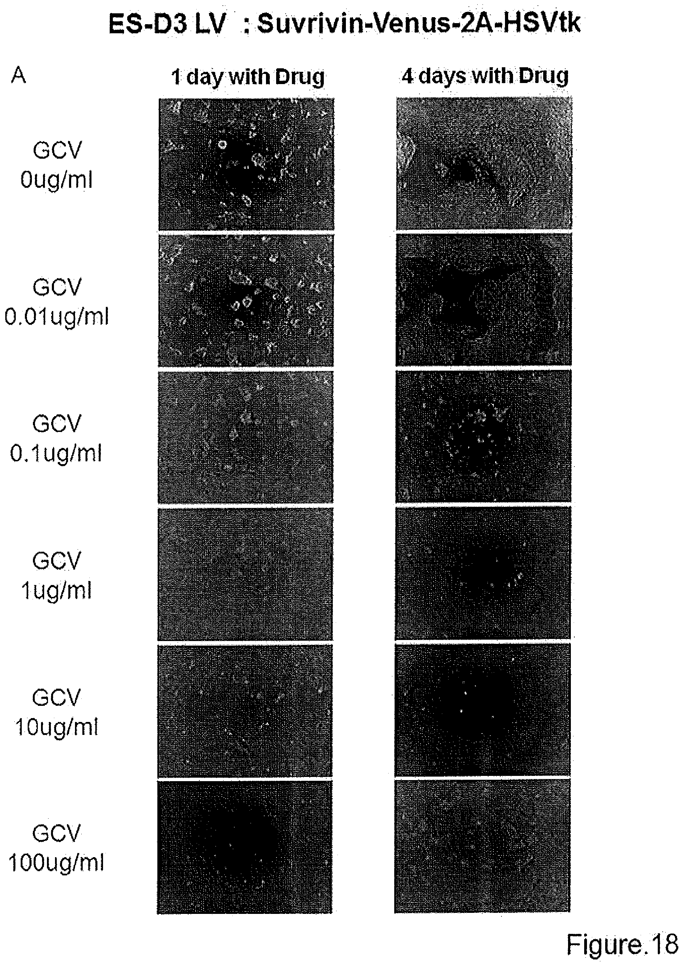

Fig. 18A shows images confirming the expression of Venus in D3 cells infected with a lentiviral vector having survivin-Venus-2A-HSV-tk. The images show the appearance of the cells on Days 1 and 4 after GCV treatment from the left.

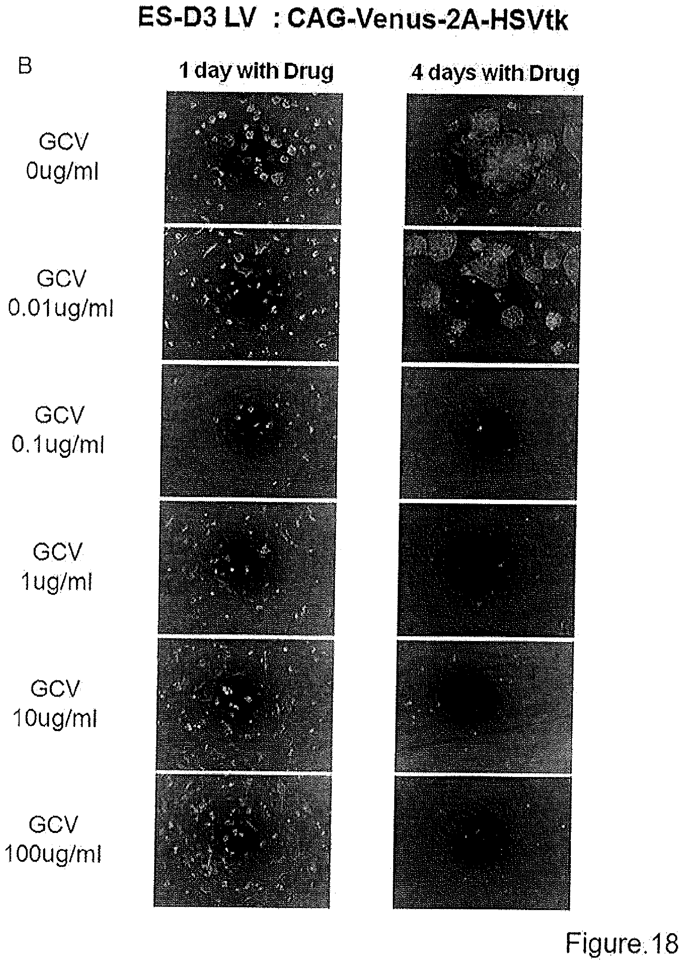

Fig. 18B shows images confirming the expression of Venus in D3 cells infected with a lentiviral vector having CAG-Venus-2A-HSV-tk. The images show the appearance of the cells on Days 1 and 4 after GCV treatment from the left.

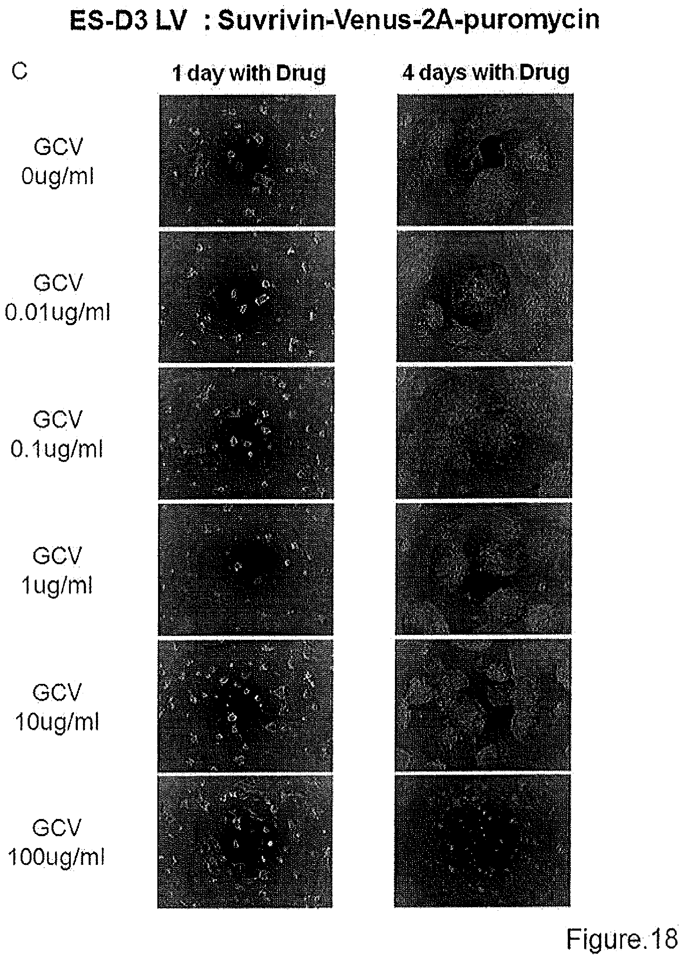

Fig. 18C shows images confirming the expression of Venus in D3 cells infected with a lentiviral vector having survivin-Venus-2A-puromycin lacking HSV-tk. The images show the appearance of the cells on Days 1 and 4 after GCV treatment from the left.

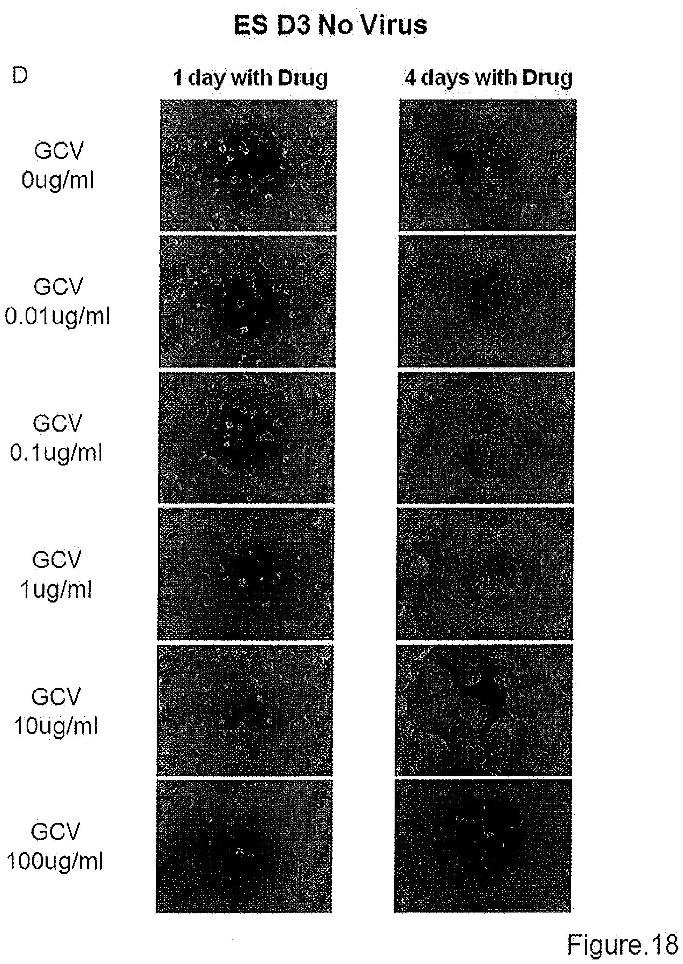

Fig. 18D shows images of D3 cells not infected with a lentiviral vector (No virus). The images show the appearance of the cells on Days 1 and 4 after GCV treatment from the left.

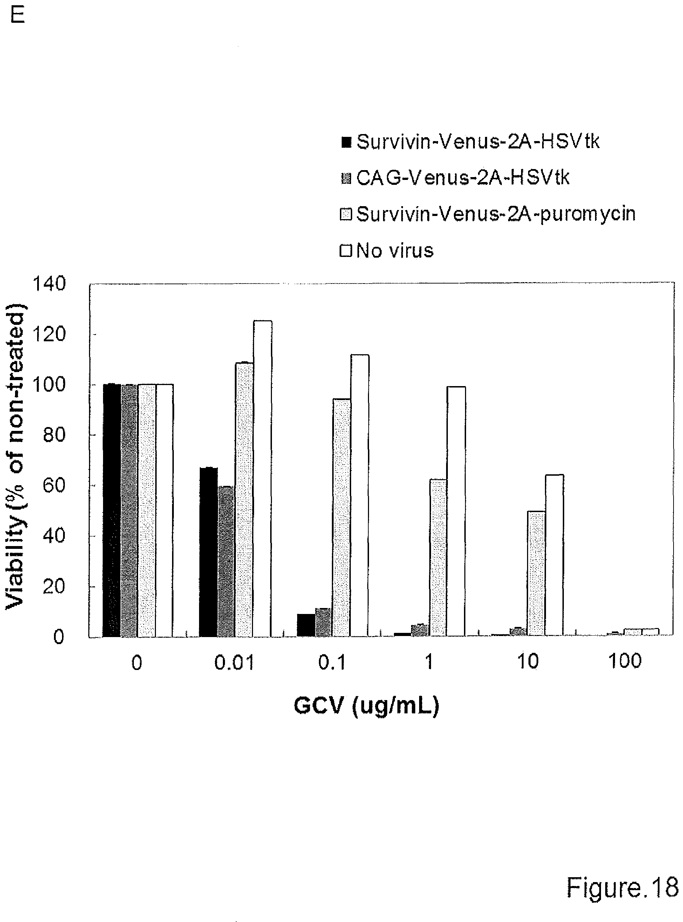

Fig. 18E shows the results of determining the viability of cells shown in Figs. 18A-18D on Day 7 after GCV treatment by WST-8 assay. The groups are the survivin-Venus-2A-HSV-tk group, the CAG-Venus-2A-HSV-tk group, the survivin-Venus-2A-puromycin group, and the non-infected D3 cell group from the left for viability after administration of GCV at each concentration.

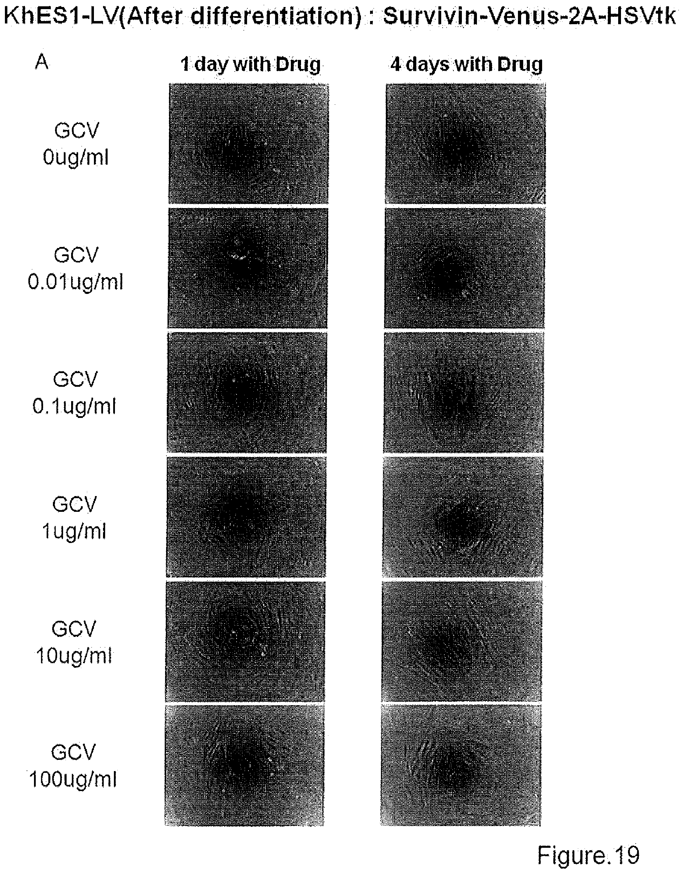

Fig. 19A show images confirming the expression of Venus in KhES1 cells after differentiation induction treatment, the cells being infected with a lentiviral vector having survivin-Venus-2A-HSV-tk. The images show the appearance of the cells on Days 1 and 4 after GCV treatment from the left.

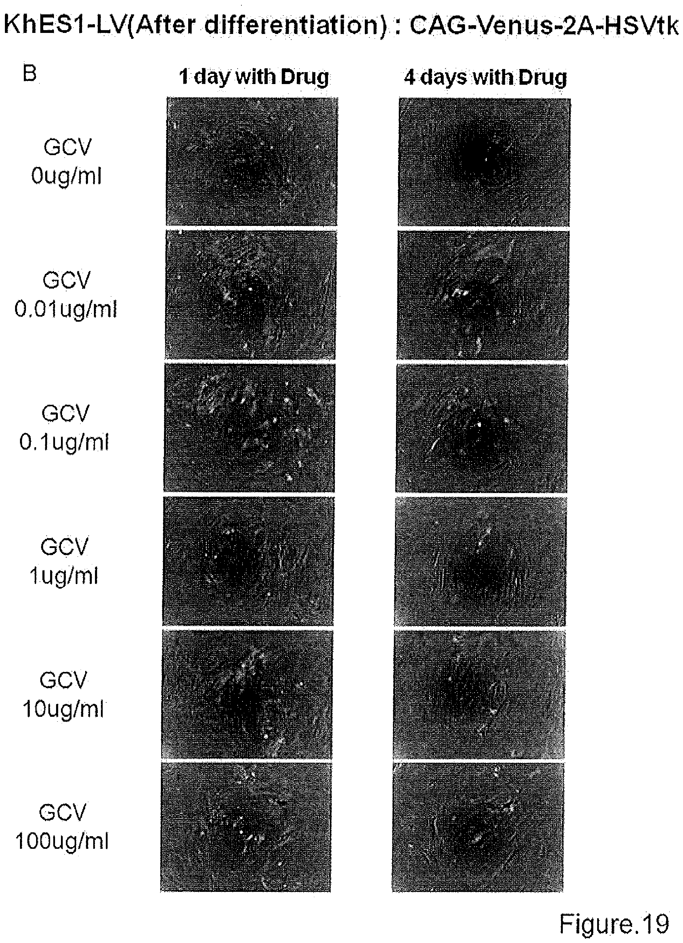

Fig. 19B show images confirming the expression of Venus in KhES1 cells after differentiation induction treatment, the cells being infected with a lentiviral vector having CAG-Venus-2A-HSV-tk. The images show the appearance of the cells on Days 1 and 4 after GCV treatment from the left.

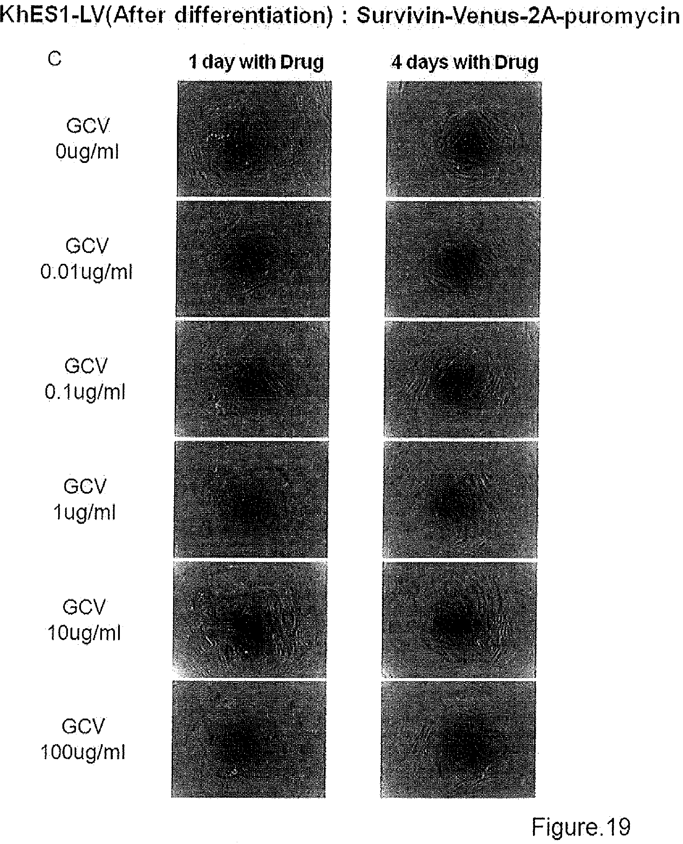

Fig. 19C show images confirming the expression of Venus in KhES1 cells after differentiation induction treatment, the cells being infected with a lentiviral vector having survivin-Venus-2A-puromycin lacking HSV-tk. The images show the appearance of the cells on Days 1 and 4 after GCV treatment from the left.

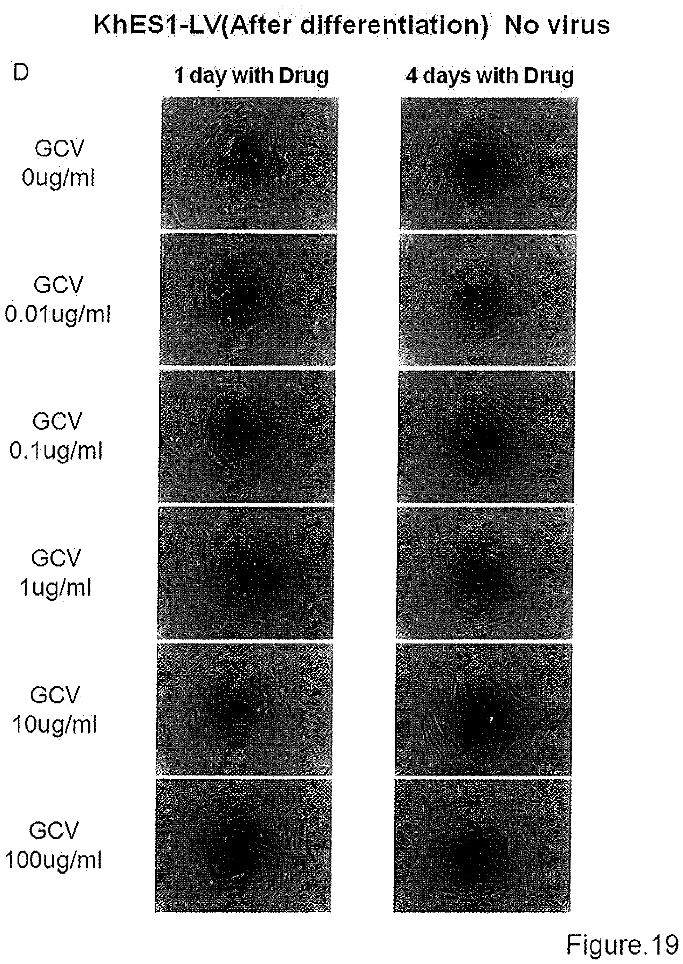

Fig. 19D show images of KhES1 cells not infected with a lentiviral vector (No virus) after differentiation induction treatment. The images show the appearance of the cells on Days 1 and 4 after GCV treatment from the left.

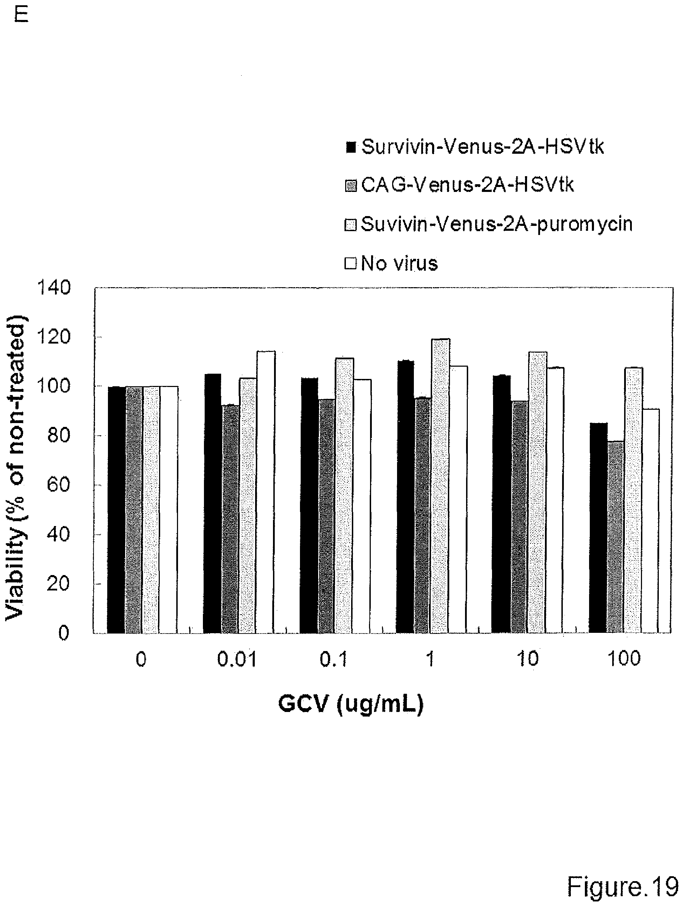

Fig. 19E shows the results of determining the viability of cells shown in Figs. 18A-18D on Day 7 after GCV treatment by WST-8 assay. The groups are the survivin-Venus-2A-HSV-tk group, the CAG-Venus-2A-HSV-tk group, the survivin-Venus-2A-puromycin group, and the non-infected KhES1 cell group from the left for viability after administration of GCV at each concentration.

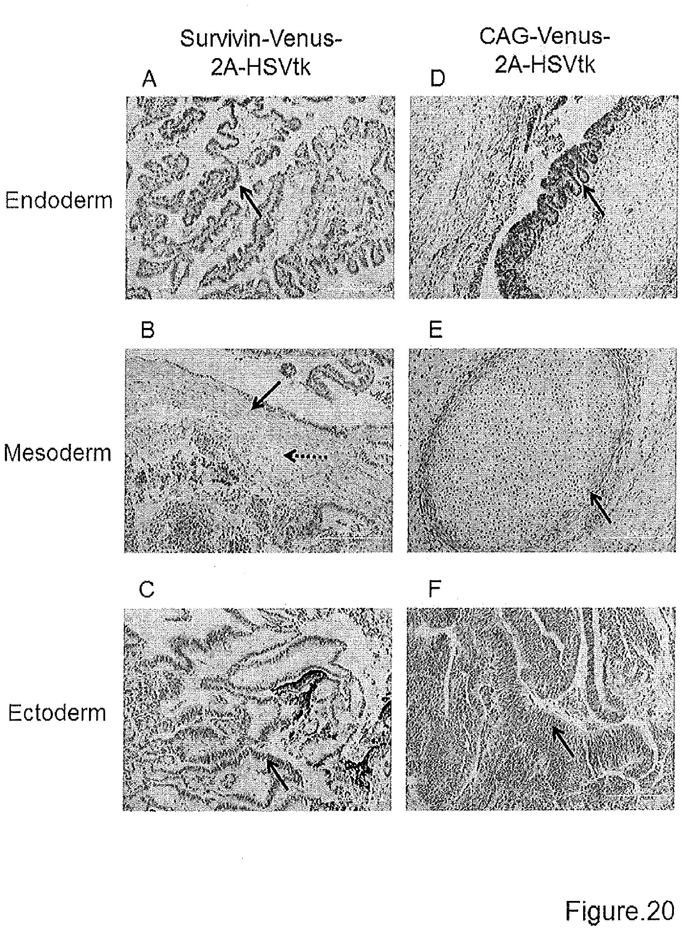

Fig. 20 shows hematoxylin-eosin (HE) staining images of teratoma sections obtained in a teratoma formation experiment. In order to confirm whether undifferentiated KhES 1 cells infected with the respective lentiviral vectors used in Fig. 17 maintain differentiation pluripotency, the cells were subcutaneously transplanted into immunodeficient mice (NOD-Scid mice). (A) to (C): It was confirmed that KhES1 cells infected with survivin-Venus-2A-HSV-tk maintain triploblastic differentiation ability as well as cells not infected with a lentivirus. Arrows represent (A) digestive tract cells (endoderm), (B) muscle cells/fat cells (broken line) (mesoderm) and (C) neural cells (ectoderm). (D) to (F): It was confirmed that KhES1 cells infected with CAG-Venus-2A-HSV-tk maintain triploblastic differentiation ability as well as cells not infected with a lentivirus. Arrows represent (D) digestive tract cells (endoderm), (E) cartilage (mesoderm), and (C) neural cells (ectoderm).

### BEST MODE FOR CARRYING OUT THE INVENTION

### 1. Production of the vector used in the method of the present invention

The vector is a viral vector as defined by the claims, containing a nucleic acid sequence in which a marker gene and a toxic gene are bound via a sequence capable of allowing a promoter to cause the two genes to be simultaneously expressed and a recombination cassette having a promoter region, in which the promoter region is operably linked to the marker gene and the toxic gene, as defined by the claims, and which can be obtained using gene recombination technology known to those skilled in the art.

### 2. Introduction of promoters

An arbitrary test promoter region or a promoter region of interest can be incorporated into the viral vector by a recombination method. Gene transfer via recombination can be performed by a method known to those skilled in the art using a shuttle vector corresponding to a gene sequence used for a recombination cassette in a vector and a recombinase.

In all methods described below, the term "cells of interest" may refer to stem cells (e.g., stem cells before or during differentiation treatment) or cells after differentiation treatment of stem cells. For example, the "cells of interest" to be infected with the viral vector correspond to target cells to be infected with the vector . That is, as long as such cells are intended to be removed or detected as undifferentiated cells left or generated after differentiation induction treatment, they may be cells obtained before or after differentiation induction. In addition, if cells of interest are stem cells obtained before or during differentiation treatment, the method may include a differentiation treatment step of inducing differentiation of cells infected with the viral vector. Further, in all methods described below, the term "cells treated for differentiation " or "cells subjected to differentiation treatment" refers to cells obtained by subjecting stem cells to differentiation treatment. Furthermore, in all methods described below, the term "undifferentiated-cell-specific labeling/killing gene expression vector" refers to the vector described above. Specifically, such vector is a viral vector containing a nucleic acid sequence in which a marker gene and a toxic gene are bound via a sequence capable of allowing a promoter to cause the two genes to be simultaneously expressed and a recombination cassette having a promoter region, in which the promoter region is operably linked to the marker gene and the toxic gene.

### 3. Method for screening for undifferentiated-cell-specific promoter

The present invention provides a method for determining activity of a test promoter in undifferentiated cells using the viral vector as defined by the claims.

In particular, the present invention relates to a method for screening for an undifferentiated-cell-specific promoter, the method comprising the steps of:
A) replacing the promoter of a lentiviral vector containing a nucleic acid sequence in which a fluorescent protein gene and a drug-dependent suicide gene are bound via a sequence capable of allowing a promoter to express the two genes simultaneously and containing a recombination cassette having the promoter region between sequences recognized by site-specific recombinases, wherein the promoter region is operably linked to the fluorescent protein gene and the drug-dependent suicide gene by a test promoter to prepare a lentiviral vector containing the test promoter;
B) infecting cells of interest with the lentiviral vector containing the test promoter;
C) detecting the expression of the fluorescent protein gene in the cells subjected to differentiation induction treatment;
D) distinguishing whether the cells subjected to differentiation induction treatment are undifferentiated cells or differentiated cells; and
E) judging whether or not the test promoter is specific to undifferentiated cells,
wherein if the expression level of the fluorescent protein gene in undifferentiated cells is higher than in differentiated cells with a statistical difference, the test promoter is determined to be specific to undifferentiated cells.

For example, the screening method may be the following method:
a method for screening for an undifferentiated-cell-specific promoter, the method comprising the steps of:
   A) replacing the promoter of a lentiviral vector containing a nucleic acid sequence in which a fluorescent protein gene and a drug-dependent suicide gene are bound via a sequence capable of allowing a promoter to express the two genes simultaneously and containing a recombination cassette having the promoter region between sequences recognized by site-specific recombinases, wherein the promoter region is operably linked to the fluorescent protein gene and the drug-dependent suicide gene by a test promoter to prepare a lentiviral vector containing the test promoter;
   B) infecting undifferentiated cells (e.g., ES cells or iPS cells) with the viral vector containing the test promoter;
   C) causing the undifferentiated cells to differentiate;
   D) detecting the expression of the fluorescent protein in the cells subjected to differentiation induction treatment;
   E) distinguishing whether the cells subjected to differentiation induction treatment are undifferentiated cells or differentiated cells; and
   F) judging whether or not the test promoter is specific to undifferentiated cells,
wherein if the expression level of the fluorescent protein gene in undifferentiated cells is higher than in differentiated cells with a statistical difference, the test promoter is determined to be specific to undifferentiated cells.

In the above method, the step of distinguishing whether the cells subjected to differentiation induction treatment are undifferentiated cells or differentiated cells can be carried out by distinguishing, as undifferentiated cells, cells positive to alkaline phosphatase staining, SSEA3 staining, SSEA4 staining, Tra-1-60 staining, and/or Tra-1-81 staining, cells expressing the Oct3/4, Nanog, Sox2, Cripto, Dax1, ERas, Fgf4, Esg1, Rex1, zfp296, UTF1, GDF3, Sall4, Tbx3, Tcf3, DNMT3L, and/or DNMT3B genes, and cells expressing miR-290 and/or miR-302 and distinguishing, as differentiated cells, cells negative to the staining or cells not expressing the gene.

According to the screening method of the present invention, when the expression level of the fluorescent protein gene in undifferentiated cells is higher than that in differentiated cells with a statistical difference, it can be judged that the rate of detection of the expression of the marker gene in cells distinguished as undifferentiated cells is "high" and the rate of detection of the expression of the marker gene in cells distinguished as differentiated cells is "low."

### 4. Infection with the vector

In the method of the present invention described above, it is possible to infect stem cells (e.g., pluripotent stem cells such as ES cells or iPS cells) before or during differentiation treatment or cells subjected to differentiation treatment with the viral vector of the present. As the vector exhibits cell toxicity in a drug-dependent manner, the vector does not exhibit cell toxicity without the addition of a drug, even if the promoter activity can be exhibited in the cells. Therefore, the order of the differentiation treatment step and the infection step would not be limited for the reason of damage to stem cells before differentiation treatment due to cytotoxicity of the introduced vector. Therefore, such features of the vector enable the determination of the best possible timing of infection in order to secure infection of all cells of interest. For example, infection is carried out *in vitro* before differentiation treatment. Alternatively, stem cells may be infected with the viral vector after differentiation treatment. Preferably, infection with the viral vector is carried out before differentiation treatment. Infection can be performed by appropriately selecting the number of viral vectors and the infection duration depending on types and properties of viral vectors to be used according to a method known to those skilled in the art. For example, infection is carried out at MOI 1 to 1000 and preferably MOI 10 to 100.

### 5. Differentiation treatment step

In view of the above, the method of the present invention may further include, for example, the step of inducing undifferentiated cells to differentiate (differentiation treatment step), which is carried out before, after, or during the step of infecting the undifferentiated cells (e.g., pluripotent stem cells such as ES cells or iPS cells) with a viral vector containing a nucleic acid sequence in which a marker gene and a drug-dependent suicide gene are bound via a 2A sequence and a recombination cassette having an undifferentiated-cell-specific promoter region, wherein the promoter region is operably linked to the marker gene and the toxic gene. Differentiation induction treatment of cells can be performed by bringing a differentiation inducer for differentiation induction into contact with undifferentiated cells and culturing the cells under appropriate culture conditions. Various culture conditions and culture methods for differentiation induction treatment have been reported, and they can be appropriately selected depending on types of cells to be used and cells that are induced to differentiate.

### 6. Detection, identification, and assay of marker genes

An expression product can be detected using a fluorescence detector, a flow cytometry device, a real-time fluorescence detector, or the like.

Hereinafter, the present invention is explained in more detail with reference to the Examples; however, the present invention is not limited thereto.

### EXAMPLES

### (Reference Example 1) Preparation of lentiviral vector plasmid

### (1) Design of lentiviral vector plasmids

Fig. 1 schematically shows a vector to be prepared. A lentiviral vector was used as a base of the vector, and a recombination cassette (RC) described below was used for the promoter region. RC is intended to perform a reaction for replacing a DNA sequence flanked by att sequences altered among vectors using the site-specific recombination system involved in the invasion of *λ* phage into an *Escherichia coli* chromosome, (James, L. et al. (2000), Genome Res. 10: 1788-1795). The RC portion has DNA sequences (attR sequences) that specifically interact with each other during recombination at both ends thereof, between which a chloramphenicol-resistant gene (CMR) and an *Escherichia coli* suicide gene (ccdB) are positioned. A lentiviral vector having RC inserted at the promoter site and a shuttle vector plasmid for gene recombination having a different promoter sequence between attL sequences corresponding to the attR sequences of RC were prepared (Fig. 1 and Fig. 2A). By using LR clonase for reacting the attR sequences of a lentiviral vector and the attL sequences of a shuttle vector, it became possible to recombine a promoter sequence with a lentiviral vector (Fig. 2B). This allowed various promoters to be conveniently changed, thereby enabling verification of activity of different promoters in undifferentiated cells/tumorigenic cells.

### (2) Fluorescent protein-2A-suicide gene

A gene cassette prepared by combining a fluorescent protein for visualizing gene transfer to cells of interest (Figs. 3 and 8) and a suicide gene for allowing a drug to selectively killing cells transfected therewith (Figs. 5 and 8) was inserted downstream of the promoter region (Figs. 1 and 7-9).

Two types of fluorescent proteins were used depending on the intended use. They were a near-infrared fluorescent protein (mKate2) having a fluorescent intensity about 100 times stronger than that of TAGFP635 and a green fluorescent protein from fluorescent jellyfish (GFP) (Venus) having improved light emission efficiency of GFP.

The following three types of suicide genes were used.

### (I) HSV-tk

It has been reported that HSV-tk has sensitivity to GCV and functions as a suicide gene (Yasuhiro TERAZAKI et al. (2003), Hepatology 37: 155-63). When HSV-tk-expressing cells are cultured in a medium containing GCV, GCV is phosphorylated by HSV-tk and further phosphorylated by endogenous kinase in human cells so that GCV is eventually formed as ganciclovir triphosphate (GCV-3P) having strong toxicity. Since GCV-3P inhibits DNA synthesis, it causes cells transfected with the HSV-tk gene to die. Meanwhile, GCV is not converted to GCV-3P in cells not expressing HSV-tk and thus does not exhibit toxicity thereto. Another advantage of this system is advanced tumor selectivity. As stated above, the cell killing action of GCV-3P is DNA synthesis inhibition. Since *in vivo* cell division does not take place or takes place to a limited extent in most of normal cells, even if HSV-tk is introduced into normal cells and expressed therein, it results in no or slight cell damage. Accordingly, tumor cells undergoing abnormal and permanent cell division are preferentially and specifically killed. It is therefore expected that when the HSV-tk gene is expressed using a tumor-specific promoter or undifferentiated-cell-specific promoter, double specificity, which consists of specificity of the promoter and specificity of HSV-tk/GCV itself, allows for the maintenance of highly advanced specificity to undifferentiated cells of tumor cells or stem cells. Yet another advantage of HSV-tk is the bystander effect on tumor cells, which is well known as the powerful tumor treatment effect that also causes most of neighboring tumor cells not transfected with HSV-tk gene to be killed. Thus, a combination of HSV-tk and GCV can strongly induce the action of killing cells undergoing active cell division (e.g., undifferentiated cells of tumor cells or stem cells) in a selective manner.

### (II) Human-tmpk/AZT

HSV-tk has highly powerful cell killing effects. However, since the origin of HSV-tk is herpes simplex, HSV-tk causes immune response in the human body, resulting in the elimination of the gene introduced into cells. This is considered problematic. Therefore, a method for combining human-derived enzymes (thymidylate kinase (tmpk) and 3'-azido-3'-deoxythymidine (AZT)) was also examined. AZT serving as a prodrug is phosphorylated so as to be converted to AZT triphosphate (AZT-3P). AZT-3P inhibits replication of human immunodeficient virus (HIV) and eukaryotic DNA synthesis (Fei CHEN et al. (2013), Biomaterials 34: 1701-11). The rate-limiting enzyme for conversion of AZT to toxic AZT-3P is cellular thymidylate kinase (tmpk) that catalyzes conversion from AZT monophosphate (AZT-MP) to AZT diphosphate (AZT-DP). Although enzymatic efficiency of tmpk is low, tmpk (F105Y) having the 200-fold increased action on AZT-MP, which is obtained via minimum gene modification, has been reported (13;14). In this experiment, this genetically improved tmpk was used. In addition, tmpk is thought to maintain all of the aforementioned advantages of HSV-tk serving as a suicide gene. It is considered that tmpk has additional advantageous features of killing tumor cells or undifferentiated cells to a greater extent and not causing heterologous-protein-derived immune induction. However, to date, there has no report on the applied use of tmpk for stem cells in any system that is not limited to the vector system of the present invention.

### (III) iCaspase9/Dimeriser

Unlike the above two genes, this gene causes cell death by inducing apoptosis. Caspase is involved in signal transduction of apoptosis. Caspase-9 involved in the early stage of the caspase cascade starting from mitochondria is usually phosphorylated by AKT or the like so as to be in the inactive state. Stimulation from mitochondria activates inactive Caspase-9 so as to activate Caspase-3 and Caspase-7, thereby inducing apoptosis. In order to control activation of Caspase-9 at an arbitrary timing, a drug-inducible Caspase-9 activation system was used. In this system, iCaspase9 (iCasp9), which is a fusion protein of the Caspase-9 gene and the human FK506-binding protein (FKBP) gene, is transferred to and expressed in cells of interest, and then, AP20187 (Clontech), which is a cell membrane-permeable low-molecular-weight compound that binds to FKBP, is added to a medium to form and activate Caspase-9dimer, thereby enabling induction of apoptosis and cell death (Carlos A Ramos et al., (2010) Stem Cells, 28: 1107-15).

### (3) Construction of lentiviral vector plasmid having HSV-tk or human-tmpk

Plasmid construction was carried out in the two stages as described in detail below. Specifically, a lentiviral vector having an inserted recombination cassette (RC) (Fig. 1A) and a fluorescent protein-2A-suicide gene cassette (Fig. 1B) were prepared, and they were eventually combined to construct a lentiviral vector plasmid (Fig. 1C).

### (3-1) Preparation of pLenti6-RC-Bls

pLenti6-V5-RC-LacZ (Invitrogen) serving as a lentiviral vector plasmid was cleaved at the Mlul site to remove the V5-LacZ region. The resulting plasmid was designated as a basic vector plasmid. After cleavage with the restriction enzyme, blunting was performed. The recombination cassette portion of a Gateway (registered trademark) plasmid (Invitrogen) was inserted to construct pLenti6-RC-Bls (Fig. 2). Various promoter sequences can be inserted at the cassette site via recombination reaction.

### (3-2) Preparation of fluorescent protein-2A-suicide gene (HSVtk/human-tmpk) cassette

A gene cassette to be inserted downstream of the RC cassette of pLenti6-RC-Bls prepared above was produced by the method described below. The gene cassette was designed to have a fluorescent protein and a suicide gene bound via a 2A sequence.

### (A) Fluorescent protein-2A

Two types of fluorescent proteins were used, which were a red fluorescent protein (mKate2) and a green fluorescent protein (Venus). pGEM-Teasy-mKate2 was constructed by designing a primer having the 5' Agel site and a primer having the 3' BglII site for mKate2 (Table 1), amplifying the primers by PCR, and cloning the amplified product into a pGEM-Teasy vector (Invitrogen) serving as a PCR product cloning vector (Fig. 3A). After cloning, sequence analysis was performed using T7 of the 5'-end primer sequence and M13R of the 3'-end primer sequence at both ends of the cloning site in the pGEM-Teasy vector. Next, mKate2 was spliced out from pGEM-Teasy-mKate2 using NotI/BglII (Fig. 4A). Synthesized double-stranded 2A sequence DNA oligo having a 5' BglII overhang and a 3' BamHI overhang was inserted into pFlag-CMV-2 (SIGMA) cleaved with BglII/BamHI so that pFlag-CMV-2A was constructed. Similarly, pFlag-CMV-mKate2-2A was constructed by cleaving pFlag-CMV-2A at NotI/BglII and inserting mKate2 between the CMV promoter and the 2A sequence (Fig. 4A).

Similarly, pGEM-Teasy-Venus was constructed by designing a primer having the 5' NotI site and a primer having the 3' BamHI site for Venus (Table 1), amplifying the primers by PCR, and cloning the amplified product into a pGEM-Teasy vector. After cloning, sequence analysis was performed using the primers. Next, Venus was spliced out at the NotI/BamHI site from pGEM-Teasy-Venus (Fig. 4B). As in the case of mKate2, Venus was inserted into pFlag-CMV-2A to construct pFlag-CMV-Venus-2A (Fig. 4B).

### (B) Suicide gene (HSV-tk, human-tmpk)

pGEM-Teasy-HSV-tk was constructed by preparing primers for HSV-tk (Table 1), amplifying the primers by PCR, and cloning the amplified product into a pGEM-Teasy vector serving as a cloning vector (Fig. 3). After cloning, sequence analysis was performed using the primers as in the cases of mKate2 and Venus. Next, pGEM-Teasy-HSVtk was cleaved at the 3' SpeI site of HSV-tk, blunted, and cleaved at the 5' BamHI site of HSV-tk so that HSV-tk was obtained (Fig. 5A).

pGEM-Teasy-human-tmpk was constructed by preparing primers for human-tmpk as in the case of HSV-tk (Table 1), amplifying the primers by PCR, and cloning the amplified product into a pGEM-Teasy vector (Fig. 3D). After cloning, sequence analysis was performed using the primers as in the case of mKate2-Venus. Next, pGEM-Teasy-human-tmpk was cleaved at the 5' BamHI site and at the 3' Smal site (blunt ends) so that human-tmpk was obtained (Fig. 5A).

### [Table 1]

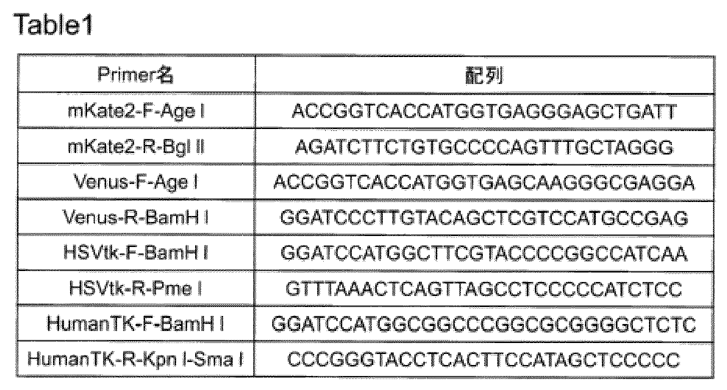

### (C) Fluorescent protein-2A+suicide gene

A vector plasmid having a fluorescent protein-2A-suicide gene expression cassette was prepared by inserting the gene fragment obtained in (B) into the plasmid prepared in (A).

pFlag-CMV-mKate2-2A and pFlag-CMV-Venus-2A were cleaved at the 3' BstXI site, blunted, and cleaved at the 5' BamHI site so as to be spliced out (Fig. 5B). HSV-tk or human-tmpk obtained in (B) was inserted at the 3' end of 2A. Accordingly, the following four types of plasmids were constructed: pFlag-CMV-mKate2-2A-HSVtk, pFlag-CMV-mKate2-2A-tmpk, pFlag-CMV-Venus-2A-HSVtk, and pFlag-CMV-Venus-2A-tmpk.

### (3-3) Preparation of a pLenti6-RC-Bls+fluorescent protein-2A-suicide gene plasmid

A monitor vector plasmid was completed by inserting a fluorescent protein-2A-suicide gene expression cassette into pLenti6-RC-Bls having an RC cassette prepared as above.

pLenti6-RC-Bls was cleaved at the 3' KpnI site and also cleaved at the 5' Agel site for ligation with a plasmid having human-tmpk (Fig. 6A). In addition, pLenti6-RC-Bls cleaved at the 3' KpnI site, blunted, and cleaved at the 5' Agel site for the use for ligation with two types of plasmids having HSV-tk (Fig. 7A).

Further, pFlag-CMV-mKate2-2A-tmpk and pFlag-CMV-Venus-2A-tmpk were cleaved at the 5' Agel site and the 3' KpnI site, and the mKate2-2A-tmpk and Venus-2A-tmpk portions were cleaved therefrom, respectively (Fig. 6B). Since HSVtk contains a KpnI cleavage site, pFlag-CMV-mKate2-2A-HSVtk and pFlag-CMV-Venus-2A-HSVtk were cleaved at 5' Agel site and the 3' PmeI site (blunt ends), and mKate2-2A-HSVtk and Venus2-2A-HSVtk were spliced out therefrom, respectively (Fig. 7B).

The above products were used in combination for ligation so that pLenti-RC-mKate2-2A-tmpk, pLenti-RC-Venus-2A-tmpk, pLenti-RC-mKate2-2A-HSVtk, and pLenti-RC-Venus-2A-HSVtk were produced.

### (4) Construction of lentiviral vector plasmid (pLenti6-iCaspase9-2A-fluorescent protein) having iCaspase9

The iCasp9-2A region was subcloned from induced-Caspase9 (a plasmid having the iCasp9-2A sequence (transferred from Dr. Brenner)) and a plasmid was constructed using an In-Fusion (registered trademark) HD cloning kit (TAKARA BIO). This kit enables cloning of DNA fragments or linearized vectors prepared by PCR with the accurate recognition of homologous 15 bases at the ends of each DNA fragment, thereby achieving rapid cloning of a plurality of DNA fragments into any vector (Zhu B. Et al. (2007), Biotechniques. 43(3): 354-9). Therefore, in order to insert iCasp9-2A-Venus or iCasp9-2A-mKate2 downstream of RC of pLenti6-RC-BLS described above, 150-bp primers were designed for both ends of iCasp9-2A and also both ends of Venus and mKate2, followed by PCR (Fig. 8, Table 2).

Thereafter, the DNA sequences of pLenti6-RC-Bls and iCasp9-2A were mixed with Venus or mKate2, thereby inducing enzymatic reaction. This led to the completion of the following two types of plasmids: pLenti6-RC-iCasp9-2A-Venus and pLenti6-RC-iCasp9-2A-mKate2.

### [Table 2]

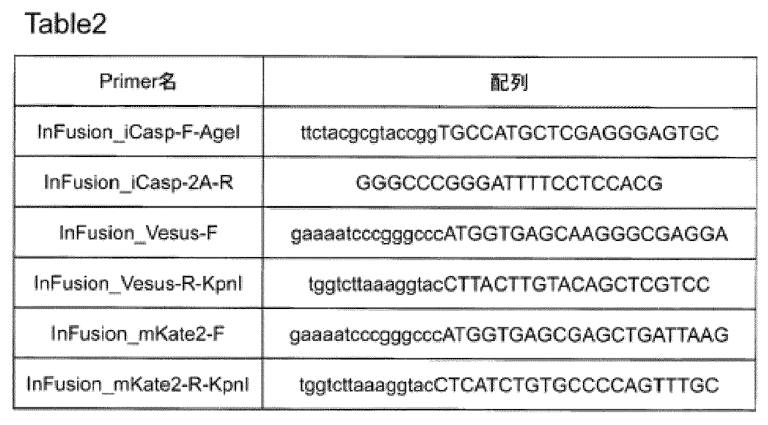

### (5) Confirmation of plasmid function

In order to confirm fluorescence expression, each plasmid having the fluorescent protein-2A-suicide gene expression cassette was introduced into HEK293 cells at the respective stages of production for evaluation. HSK293 cells were seeded on a 6-well plate at 6 × 10⁵ cells/well. On the following day, gene transfer was performed using a Polyfect Transfection Reagent Kit (QIAGEN) according to the protocol provided by the manufacturer with the addition of each plasmid at 2 µg/well. Fluorescence expression was confirmed 48 hours after gene transfer using a fluorescence microscope.

### (6) Results

pFlag-CMV-mKate2-2 and pFlag-CMV-Venus-2A were introduced into HEK293 to evaluate fluorescent protein expression. As a result, mKate2 and Venus were confirmed to be normally expressed under the control of CMV serving as a constitutive promoter (Fig. 9). Similarly, pFlag-CMV-mKate2-2A-HSVtk, pFlag-CMV-Venus-2A-HSVtk, pFlag-CMV-mKate2-2A-Human tmpk, and pFlag-CMV-Venus-2A-Human tmpk were also introduced into HEK293 cells to evaluate fluorescent protein expression and suicide gene expression. As a result, mKate2 and Venus were confirmed to be normally expressed (Figs. 10A and 10C). Further, drugs corresponding to the respective suicide genes were added. As a result, cytotoxic effects were observed in a drug-concentration-dependent manner (Figs. 10A to 10D).

### (Reference Example 2) Verification of cytotoxic effects of suicide genes on cultured human cells

In order to verify cytotoxic effects of suicide genes on cultured human cells, the cells were infected with viral vectors capable of expressing the three different suicide genes (HSV-tk, human-tmpk, and iCaspase9). Then, drugs corresponding to the respective suicide genes were added to compare cytotoxic effects on the cells. The preliminarily prepared adenoviral vector was used for HSV-tk (Yasuhiro Terazaki et al., (2003), Hepatology 37; 155-63). For human-tmpk and iCaspase9, the following plasmids were respectively used: pHR'-cPPT-EF-Tmpk (F105Y) (Takeya Sato et al. (2007), Mol Ther 15: 962-70) transferred from Dr. Medin; and iCasp9-2A-ΔCD19RRF (CARLOS A RAMOS et al. (2010), STEM Cells 28; 1107-15) transferred from Dr. Brenner. These plasmids were directly introduced into cells or lentiviral vectors were produced and used for the plasmids.

### (1) Cell culture

201B7 established by introducing four factors derived from adult human dermal fibroblasts (HDF) (OCT3/4, KLF4, SOX2 and c-Myc) and 253G1 established by introducing three HDF-derived factors other than c-Myc were used as human iPS cells. In addition, D3 cells were used as mouse ES cells. KhES1 cells (transferred from Kyoto University) were used as human ES cells. In addition, human normal fibroblasts (BJ) and human cancer cells (human T lymphocyte cell line: Jurkat; and human gastric cancer cell line: MKN45) were used. Human iPS/ES cells were cocultured on mouse embryonic fibroblasts (MEF) treated with mitomycin C. Mouse ES cells were cultured on a gelatin-coated dish. Media used for culture of the cells are described below.

### (Human iPS/ES cells)

20% KSR (Invitrogen), 200 mM L-Glutamine, 0.1 mM Non Essential Amino Acid (Sigma), 0.1 mM 2-Mercaptoethanol (Sigma), 5 ng/mL Basic-Fgf (Wako), DMEM-F12 (Sigma) containing Penicillin/Streptomycin (P/S) (Nacalai Tesque)

### (Mouse ES cells)

20% Fetal Bovine Serum (FBS) (GIBCO), 0.1 mM Non Essential Amino Acid, 1 mM Sodium Pyruvate (Gibco), 70 µM 2-Mercaptoethanol, 1 × 10³ unit/mL LIF mouse recombinant (Nacalai Tesque), P/S-containing DMEM (SIGMA)

### (Jurkat cells and MKN45 cells)

10% FBS (Bio West) and P/S-containing RPMI-1640 (SIGMA)

### (BJ cells)

10% FCS, 200 mM L-Glutamine (Gibco), 0.1 mM Non Essential Amino Acid, 1 mM Sodium Pyruvate, P/S-containing Minimum Essential Medium Eagle (Sigma)

Undifferentiated human iPS and ES cells used for viral infection experiments were cultured by feeder-free diffusion culture. MEF medium (1.5 × 10⁶ cells/10 CM dish) treated by mitomycin C was replaced by human ES cell medium (BFGF-), followed by overnight culture in an incubator at 37°C. The supernatant was used as a conditional medium (CM) for feeder-free diffusion culture.

Single-cell diffusion culture of KhES1 was carried out using a ROCK inhibitor (Y27632, WAKO) under feeder-free conditions and the culture was used for viral infection experiments. Growth Factor Reduced BD Matrigel (BD Bioscience) that had been diluted 40 folds with serum-free medium (DMEM-F12) was added to a 24-well plate. The plate was allowed to stand still in a CO₂ incubator at 37°C for 1 hour for Matrigel coating treatment. The medium for KhES1 in the 6cm dish was replaced by fresh medium (2 mL), and Y27632 was added to result in a final concentration of 10 µM, followed by culture in a CO₂ incubator at 37°C for 1 hour. Y27632-treated human ES cells were washed with 2 mL of PBS, 0.4 mL of a cell dissociation solution for human ES cells (CTK) was added, and the plate was allowed to stand still at 37°C for 5 minutes. Then, 2 mL of 10 µM Y27632-containing human ES cell medium was added, and pipetting was performed so that cell mass was dispersed into separate cells. The number of cells was counted. A necessary amount of KhES1 cells were centrifuged (1000 rpm, 5 minutes), suspended in CM, and seeded on a Matrigel-coated plate.

### (2) Production of lentivirus

pHR-CPPT-EF-tmpk (for human-tmpk expression), iCasp9.2A.ACD19.RRF (for iCasp9 expression), and CMV-EGFP were used as lentiviral vectors. Since pHR-CPPT-EF-tmpk and iCasp9.2A.ACD19.RRF express CD19 and a suicide gene at the same time, the expression of each suicide gene can be confirmed using an anti-CD19 antibody. CMV-EGFP was used for confirming the titer of lentiviral vectors.

HEK293T cells were seeded at 6 × 10⁵ cells/well on a 6-well plate coated with POLY-L-LYSINE (SIGMA). On the following day, pLP1, pLP2, and pLP-VSVG were mixed with 0.5 µg of each lentiviral vector plasmid and 50 µL of OPTI-MEM was added. Then, 6 µL of FUGENE HD (PROMEGA) was added, followed by vortexing and stationary placement at room temperature for 15 minutes. The total amount of the resulting product was added to 1 well for transfection. The medium was replaced by 10% FCS/DMEM, P/S(-) 24 hours later. The supernatant was collected 48 and 72 hours later (6-well plate: collection of 2 mL × 4 wells for two times, 16 mL in total). Each supernatant was 100-fold concentrated using a Lenti-X Concentrator Kit (Clonetech) according to the protocol provided by the manufacturer.

### (3) Lentiviral infection

On the day before infection, cells of each type described below were seeded on a 24-well plate at the corresponding number of cells.

KhES1 cells: 4 × 10⁴ cells/well
Jurkat cells: 1.25 × 10⁵ cells/well
MKN45 cells: 2 × 10⁴ cells/well
HEK293T cells: 5 × 10⁴ cells/well

On the day of viral infection, the medium was replaced by medium containing 4 µg/mL polybrene. Each lentiviral vector (20 µL) was added for infection for 24 hours. The medium for the cells of each type was renewed 24 hours later, and culture was further continued. Cells infected with the EGFP-expressing lentiviral vector were evaluated 72 hours after infection using a flow cytometer (Cyan Adp Analyzer, Beckman Coulter), and the titer of the produced viral vector was calculated.

### (4) Fractionation of CD19-expressing cells by flow cytometry analysis (FACS)

Regarding cells infected with human-tmpk-expressing lentiviral vectors, infected cells (CD19-positive cells) were sorted using FACS ARIA II (BD).

For KhES1, KhES1 cells cultured overnight in medium containing 10 µM Y27632 was treated with CTK so that the cells were dispersed into separate cells. After counting of the number of cells, a necessary amount of cells were centrifuged (1000 rpm, 5 minutes) and resuspended in 10 µM Y27632-containing PBS. Alexa Fluor 488 anti-Human SSEA4 (BIOLEGEND) and anti-Human CD19-APC (EBIOSCIENCE), which are antibodies for labeling undifferentiated cells, were added (5 µL each) to 100 µL of the cell suspension (1 × 10⁶ cells), and cells were suspended on ice for every 10 minutes during stationary placement for 30 minutes for labeling of the cells. The cells were washed once with 10 µM Y27632-containing PBS, suspended in 10 µM Y27632-containing ES medium, and then KhES1 cells labeled with both antibodies were evaluated as undifferentiated CD19-positive cells using a flow cytometer.

For Jurkat and MKN45, after counting of the number of cells, a necessary amount of cells were centrifuged (1000 rpm, 5 minutes) and resuspended in 10% FCS-containing PBS. Anti-Human CD19-APC (EBIOSCIENCE) (5 µL) was added to 100 µL of the cell suspension (1 × 10⁶ cells), and cells were suspended on ice for every 10 minutes during stationary placement for 30 minutes for labeling of the cells. The cells were washed once with 10% FCS-containing PBS, suspended in 10% FCS-containing PBS, and then labeled Jurkat and MKN45 cells were evaluated as CD19-positive cells using a flow cytometer.

After confirmation of CD19-positive cells using a flow cytometer, cells were increased and labeled again with antibodies according to the above protocol. Then, positive cells were sorted using BD FACSARIA II. SSEA4- and CD19-positive cells of KhES1 and CD19-positive cells of Jurkat and MKN45 were sorted. Fractionated cells were immediately centrifuged (1000 rpm, 5 minutes) and resuspended in fresh medium. KhES 1 cells were cocultured with MEF cells, and Jurkat and MKN45 were cultured in a usual manner.

### (5) Verification of in vitro cytotoxic effects

In order to evaluate cytotoxicity of suicide genes in cells of each type, HSV-tk, human-tmpk, and iCaspase9 were introduced into cells, and cell killing effects were examined and compared with the addition of drugs. For HSV-tk, the preliminarily prepared HSV-tk adenovirus (Ad.CAG-HSV-tk) was used. For human-tmpk and iCasp9, plasmids were introduced into cells. Cells of each type described below were seeded on a 24-well plate at the corresponding number of cells.

KhES1 cells: 4 × 10⁴ cells/well
MKN45 cells: 2 × 10⁴ cells/well (only tmpk iCasp9)
BJ cells: 1 × 10⁴ cells/well

On the following day, infection with Ad.CAG-HSV-tk at MOI = 10 was performed, the medium was renewed 24 hours later, and then 10 µL of GCV (10 µg/mL) was administered for 4 days. For human-tmpk and iCasp9, 0.5 µg of each plasmid was added to 25 µL of OPTI-MEM, and 2 µL of FUGENE HD (QIAGEN) was added, followed by vortexing and stationary placement of 15 minutes. The total amount of the resulting product was administered. The medium was renewed 24 hours later. Then, AZT was administered at 0, 30, 100, and 300 µM to each well of human-tmpk-transfected cells for 3 days. For iCasp3, Dimerizer was administered at 0, 0.03, 0.1, and 0.3 µM to each well for 3 days. After drug administration, cytotoxic effects were determined by counting the number of viable cells. On the day of determination, KhES1 cells were treated with CTK so that the cells were dispersed into separate cells. Then, 10 µL of the cells were collected. For Jurkat, 10 µL of the cell suspension was collected. MKN45 was treated with trypsin and 10 µL of the cell suspension was collected. Trypan blue (10 µL) was added to each collected cell suspension and the number of viable cells was counted.

In addition, regarding human-tmpk, after infection with a lentiviral vector, a similar experiment was conducted using sorted cells permanently-expressing the gene based on specificity to CD19. Cells of each type described below were seeded on a 24-well plate at the corresponding number of cells.

Jurkat-tmpk/NC: 1 × 10⁵ cells/well
MKN45 cells -tmpk/NC: 2 × 10⁴ cells/well

Starting from the following day, AZT was administered at 0, 0.1, 1, 10, 100, and 1000 µM to each well for 3 days, and the number of viable cells was counted by the method described above.

### (6) Results

Ad.CAG-HSV-tk-infected KhES1 cells used as a control were damaged due to GCV administration, while on the other hand, such damage was not observed in the no-GCV-treatment group (Fig. 11A). No damage was observed for BJ of the no-GCV-treatment group, and cell damage due to GCV administration was slightly observed compared with KhES1 (Fig. 11B).

Cytotoxic effects were confirmed in KhES1 cells transfected with pHR-CPPT-EF-tmpk (F105Y) even after low-concentration AZT administration. Cell-killing effects (Fig. 12A) were comparable to those observed in the case of HSV-tk transfection with the adenoviral vector (Fig. 11A). As a result of high-concentration AZT administration, cell damage was observed even in cells not transfected with human-tmpk. There was no difference in cell damage for BJ between human-tmpk-transfected cells and non-transfected cells (Fig. 12B). Cytotoxic effects were confirmed for MKN45 cells as well as undifferentiated cells even after low-concentration AZT administration. Cell damage was observed even in cells not transfected with human-tmpk after high concentration AZT administration (Fig. 12C). However, there was substantially no change in KhES1, BJ, and MKN45 cells transfected with iCasp9.2A.ACD19.RRF.

Regarding human-tmpk which had effects on drug sensitivity as a result of transient expression of the plasmid, a cell line permanently expressing human-tmpk was produced and analysis was further performed. A lentiviral vector expressing human-tmpk and CD19 was produced from pHR-CPPT-EF-tmpk (F105Y). Cells were infected with the vector and sorted based on specificity to CD19. KhES1, Jurkat, and MKN45 cells were sorted and grown, and then the cells were analyzed again with an anti-CD 19 antibody using a flow cytometer. As a result, CD19-positive cells were found to be present at a higher proportion than that before sorting (Figs. 14A to 14C). AZT was administered to these infected cells. Accordingly, cytotoxic effects on Jurkat tended to increase in a drug-dependent manner. At high concentrations, cell damage was observed even in cells not transfected with human-tmpk (Figs. 15A and 15C). Such concentration-dependent cell killing effects were not observed in MKN45, compared with Jurkat, and cell damage was induced at high concentrations even in cells not infected with human-tmpk (Figs. 15B and 15C).

### (Reference Example 3) Verification of cytotoxic effects of suicide genes in human cultured cells using the prepared lentiviral vector plasmid

In order to verify cytotoxic effects of suicide genes in human cultured cells, various promoters were inserted into the lentiviral vector plasmid containing RC prepared in Example 1 via recombination so that plasmids were completed. Lentiviral vectors were produced using these plasmids so as to infect cells. Then, drugs were added to the corresponding lentiviral vectors for the comparison of cytotoxic effects on cells of each type.

### (1) Subcloning of promoter into shuttle vector plasmid

Various promoters were inserted into a pENTR-vector (LIFE TECHNOLOGIES) (Fig. 15). The pENTR-vector has attL sequences. It is used as a shuttle vector that is reacted with pLenti6-RC using LR clonase so that a promoter is incorporated into RC. Regarding promoters used for subcloning, survivin, tert, Rex1, and Nanog were selected as undifferentiated-cell-specific promoter candidates. In addition, constitutive promoters (CAG and PGK) were used as controls. Each promoter was inserted into a pENTR-vector by the corresponding method described below.

### (A) Survivin promoter

The survivin promoter region was spliced out using NotI from the preliminarily prepared pGEMT-easy-survivin and cloned into a pENTR-vector so that pENTR-survivin was constructed (Fig. 15A).

### (B) Tert promoter

The tert promoter region was spliced out using MluI/BglII from the preliminarily prepared pGEMT-easy-tert and cloned into a pENTR-vector so that pENTR-Tert was constructed (Fig. 15B).

### (C) Rex1 promoter

The Rex1 promoter region was spliced out using MunINheI from the preliminarily prepared pGEMT-easy-Rex1 and cloned into a pENTR-vector so that pENTR-Rex1 was constructed (Fig. 15C).

### (D) Nanog promoter

pENTR-Nanog was constructed by preparing primers each having a CACC overhang for pENTR-vector cloning for the 3' and 5' ends of the Nanog promoter region (Table 3), amplifying the primers by PCR, and cloning the amplified product into a pENTR-vector (Fig. 15D)

### (E) CAG promoter

The CAG promoter region was spliced out using SalI/EcoRI (blunting) from the preliminarily prepared pCX-EGFP and cloned into a pENTR-vector so that pENTR-CAG was constructed (Fig. 15E).

### (F) PGK promoter

pENTR-PGK was constructed by preparing a primer having a CACC overhang for pENTR-vector cloning and a primer having the PstI site (Table 3) for the 5' and 3' ends of the PGK promoter region, respectively, amplifying the primers by PCR, and cloning the amplified product into a pENTR-vector (Fig. 15F)

### [Table 3]

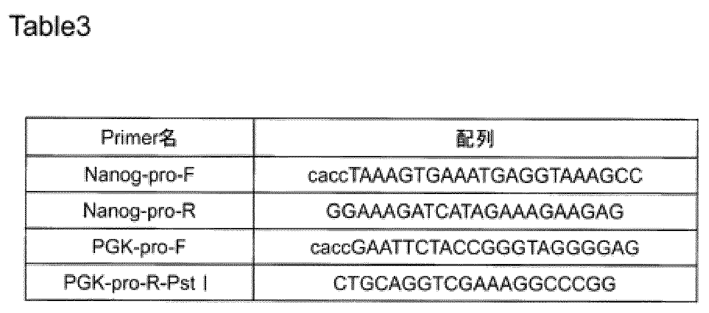

### (2) Promoter recombination in lentiviral vector plasmid containing RC

Among the plasmids shown in Fig. 7, various promoters shown in Fig. 15 were inserted via recombination into the RC portion of pLenti6-RC-Venus-2A-HSV-tk (pLenti6-Promoter-Venus-2A-HSV-tk). A lentiviral vector plasmid containing RC and a pENTR vector plasmid containing each promoter were mixed and reacted with LR Clonase so that the promoter was inserted into the RC portion. Survivin, tert, Rex1, and Nanog were inserted as undifferentiated-cell-specific promoters, and constitutive promoters (CAG and PGK) were inserted as controls (Fig. 16A). In addition, among the plasmids shown in Fig. 7, various promoters shown in Fig. 15 were inserted into the RC portion of pLenti6-RC-Venus-2A-human-tmpk via recombination (pLenti6-Promoter-Venus-2A-human-tmpk). Survivin, tert, Rex1, and Nanog were inserted as undifferentiated-cell-specific promoters, and constitutive promoters (CAG and PGK) were inserted as controls (Fig. 16B).

### (3) Fractionation of GFP-expressing cells by flow cytometry analysis (FACS)

Among the plasmids (pLenti6-Promoter-Venus-2A-HSV-tk), plasmids containing survivin and CAG promoters were used for producing lentiviral vectors. Cells were infected with the vectors. The infected cells (GFP-positive cells) were sorted using FACS ARIA II (BD).

In the case of KhES1, KhES1 cells were cultured overnight in medium containing 10 µM Y27632, treated with CTK, and dispersed into separate cells. The number of cells was counted, a necessary amount of cells were centrifuged (1000 rpm, 5 minutes), and resuspended in 10 µM Y27632-containing PBS. Alexa Fluor 488 anti-Human SSEA4 (BIOLEGEND) serving as an antibody for labeling undifferentiated cells (5 µL) was added to 100 µL of a cell suspension (1 × 10⁶ cells), cells were suspended on ice for every 10 minutes during stationary placement for 30 minutes for labeling of the cells. The cells were washed once with 10 µM Y27632-containing PBS, suspended in 10 µM Y27632-containing ES medium, and then GFP-positive cells labeled with the antibody were evaluated as undifferentiated lentivirus-infected KhES1 cells using a flow cytometer.

D3 cells was treated with trypsin, and pipetting was performed so that cell mass was dispersed into separate cells. The number of cells was counted. A necessary amount of D3 cells were centrifuged (1000 rpm, 5 minutes) and resuspended in medium. GFP-positive cells were evaluated as lentivirus-infected D3 cells using a flow cytometer.

GFP-positive cells were confirmed using a flow cytometer and the cells were grown. Then, GFP-positive cells were sorted using BD FACSARIA II. Fractionated cells were immediately centrifuged (1000 rpm, 5 minutes) and resuspended in fresh medium, KhES 1 cells were cocultured with MEF cells, and D3 cells were seeded on a gelatin-coated dish.

### (4) Verification of effects of in vitro toxicity on undifferentiated cells

Cytotoxic effects after GCV administration were analyzed using the above sorted KhES1 cells and D3 cells. KhES1 cells were seeded on a 96-well plate at 1 × 10⁵ cells per well. Starting from the following day, GCV was administered at 0, 0.01, 0.1, 1, 10, and 100 µg/mL to the groups of survivin-Venus-2A-HSV-tk (A), CAG-Venus-2A-HSV-tk (B), survivin-Venus-2A-puromycin lacking HSV-tk (C), and non-infected cells (NC) (D) for 7 days. Fig. 17 shows the results. D3 cells were seeded on a 96-well plate at 1 × 10⁵ cells per well. Starting from the following day, GCV was administered at 0, 0.01, 0.1, 1, 10, and 100 µg/mL to the groups of survivin-Venus-2A-HSV-tk (A), CAG-Venus-2A-HSV-tk (B), survivin-Venus-2A-puromycin lacking HSV-tk (C), and non-infected cells (NC) (D) for 4 days. Cytotoxic effects after 4-day administration were determined by WST-8 assay, which is modified MTT assay. The reagent SF (Nacalai Tesque) used herein is a trazolium salt that produces highly sensitive water-soluble formazan; the use of WST-8 [2-(20methoxy-4-nitrophenyl)-3-(nitrophenyl)-5-(2,4-disulgophenyl)-2H-tetrazolium monosodium salt] as a chromogenic substrate allows improved high-sensitivity assay, compared with conventionally used MTT, and WST-8 is reduced by intracellular dehydrogenase, thereby producing water-soluble formazan. Absorbance of this formazan at 450 nm was directly measured, thereby determining the number of viable cells (E). Fig. 18 shows the results.

### (5) Results

Sufficient cytotoxic effects were observed in KhES1 cells infected with LV.survivin-Venus-2A-HSV-tk as well as LV.CAG used as a control at a low GCV dose of 1 µg/mL, resulting in death of almost all of the cells (Fig. 17A,B). Meanwhile, cytotoxic effects were not observed in cells infected with LV.survivin-Venus-2A-puromycin lacking HSV-tk and non-infected cells (NC) at the same dose (Fig. 17C,D), indicating that specific cytotoxic effects dependent on HSV-tk were confirmed upon administration of GCV at 1 µg/mL. In addition, at a high GCV concentration of 10 µg/mL or more, HSV-tk-non-specific cytotoxicity was observed. Further, similar results were also obtained for D3 cells. In particular, cytotoxic effects on D3 cells infected with LV.survivin-Venus-2A-HSV-tk were confirmed with higher sensitivity than that for KhES1 cells infected with the same virus (Figs. 18A-18E).

### (6) Verification of safety upon in vitro GCV administration to differentiated cells

Analysis was conducted using the above sorted KhES1 cells to confirm safety upon GCV administration after differentiation induction. For differentiation induction, cells were seeded on a 96-well low adhesive plate containing ES medium (excluding LIF) at 3 × 10³ cells per well. Culture was performed for one week so that an embryonic body was formed. Thereafter, subculture on a gelatin-coated dish was continued for 28 days or more so that sufficiently differentiated cells were produced. Cells subjected to differentiation induction were re-seeded on a 96-well plate at 1 × 10⁵ cells per well. Starting from the following day, GCV was administered at 0, 0.01, 0.1, 1, 10, and 100 µg/mL to the groups of survivin-Venus-2A-HSV-tk (A), CAG-Venus-2A-HSV-tk (B), survivin-Venus-2A-puromycin lacking HSV-tk (C), and non-infected cells (NC)(D) for 4 days. Fig. 19 shows the results.

### (7) Results

Cytotoxic effects were observed in undifferentiated KhES1 cells infected with LV.survivin-Venus-2A-HSV-tk as well as LV.CAG used as a control. However, substantially no damage was observed in differentiated cells obtained by the above method. Undifferentiated-cell-specific-killing effects were confirmed (Figs. 19A and 19B). No cytotoxicity was observed in cells infected with LV.survivin-Venus-2A-puromycin lacking HSV-tk and non-infected cells (NC) as well as undifferentiated cells (Figs. 19C and 19D). Non-specific cell killing effects were not observed even upon administration of GCV at 10 µg/mL or more, compared with undifferentiated cells. Accordingly, it was found that GCV exhibits toxicity only in undifferentiated cells under the presence of HSV-tk (Figs. 19A-19E).

### (8) Verification of in vivo differentiation pluripotency in lentivirus-infected human ES cells

Verification was conducted using the above sorted KhES1 cells to confirm the assumption that lentivirus infection would not influence differentiation pluripotency. The cells (4 × 10⁶ cells) were subcutaneously transplanted in immunodeficient mice so that teratoma was formed. Each teratoma with a diameter of 5 mm or more was removed and fixed with formalin. Then, paraffin-embedded sections were prepared. Each section was deparaffinized, followed by HE staining. Fig. 20 shows the results.

### (9) Results

Triploblastic-derived cells were observed among both KhES1 cells infected with LV.survivin-Venus-2A-HSV-tk and KhES1 cells infected with LV.CA-Venus-2A-HSV-tk (Figs. 20A-20F), suggesting that the cells maintained pluripotency to an extent comparable to that of non-infected cells. The results confirmed that lentivirus infection does not influence differentiation pluripotency of pluripotent stem cells, as previously reported in many studies.

## Claims

1. An *in-vitro* method for screening for an undifferentiated-cell-specific promoter, the method comprising the steps of:
A) replacing the promoter of a lentiviral vector containing a nucleic acid sequence in which a fluorescent protein gene and a drug-dependent suicide gene are bound via a sequence capable of allowing a promoter to express the two genes simultaneously and containing a recombination cassette having the promoter region between sequences recognized by site-specific recombinases, wherein the promoter region is operably linked to the fluorescent protein gene and the drug-dependent suicide gene by a test promoter to prepare a lentiviral vector containing the test promoter;
B) infecting cells of interest with the lentiviral vector containing the test promoter;
C) detecting the expression of the fluorescent protein gene in said infected cells subjected to differentiation induction treatment;
D) distinguishing whether said infected cells subjected to differentiation induction treatment are undifferentiated cells or differentiated cells; and
E) judging whether or not the test promoter is specific to undifferentiated cells,
wherein if the expression level of the fluorescent protein gene in undifferentiated cells is higher than in differentiated cells with a statistical difference, the test promoter is determined to be specific to undifferentiated cells.

2. The method according to claim 1, wherein the sequence capable of allowing a promoter to cause the two genes to be simultaneously expressed is 2A sequence.

3. The method according to claim 1 or 2, wherein the drug-dependent suicide gene is HSV-tk, human-tmpk, a cytosine deaminase gene, or caspase.

4. The method according to claim 3, wherein the drug-dependent suicide gene is HSV-tk.

5. The method according to any one of claims 1-4, wherein the fluorescent protein is a red fluorescent protein or green fluorescent protein.

6. The method according to claim 5, wherein the fluorescent protein is mKate2 or Venus.

## Patentansprüche

1. In-vitro-Verfahren zum Screening auf einen undifferenzierten zellspezifischen Promotor, wobei das Verfahren die folgenden Schritte umfasst:
A) Ersetzen des Promotors eines lentiviralen Vektors, enthaltend eine Nukleinsäuresequenz, in der ein fluoreszierendes Proteingen und ein arzneimittelabhängiges Suizidgen über eine Sequenz gebunden sind, die in der Lage ist, es einem Promotor zu ermöglichen, die beiden Gene gleichzeitig zu exprimieren, und enthaltend eine Rekombinationskassette mit der Promotorregion zwischen Sequenzen, die von ortsspezifischen Rekombinasen erkannt werden, wobei die Promotorregion mit dem fluoreszierenden Proteingen und dem arzneimittelabhängigen Suizidgen durch einen Testpromotor funktionsfähig verbunden wird, um einen lentiviralen Vektor herzustellen, der den Testpromotor enthält;
B) Infizieren von Zellen von Interesse mit dem lentiviralen Vektor, der den Testpromotor enthält;
C) Nachweis der Expression des fluoreszierenden Proteingens in den infizierten Zellen, die einer Differenzierungsinduktionsbehandlung unterzogen wurden;
D) Unterscheidung, ob die infizierten Zellen, die einer Differenzierungsinduktionsbehandlung unterzogen wurden, undifferenzierte Zellen oder differenzierte Zellen sind; und
E) Beurteilung, ob der Testpromotor spezifisch für undifferenzierte Zellen ist oder nicht, wobei, wenn die Expressionsstärke des fluoreszierenden Proteingens in undifferenzierten Zellen höher ist als in differenzierten Zellen mit einem statistischen Unterschied, der Testpromotor als spezifisch für undifferenzierte Zellen bestimmt wird.

2. Verfahren nach Anspruch 1, wobei die Sequenz, die in der Lage ist, es einem Promotor zu ermöglichen, zu bewirken, dass die beiden Gene gleichzeitig exprimiert werden, die 2A-Sequenz ist.

3. Verfahren nach Anspruch 1 oder 2, wobei das arzneimittelabhängige Suizidgen HSV-tk, Human-tmpk, ein Cytosindeaminasegen oder Caspase ist.

4. Verfahren nach Anspruch 3, wobei das arzneimittelabhängige Suizidgen HSV-tk ist.

5. Verfahren nach einem der Ansprüche 1-4, wobei das fluoreszierende Protein ein rot fluoreszierendes Protein oder ein grün fluoreszierendes Protein ist.

6. Verfahren nach Anspruch 5, wobei das fluoreszierende Protein mKate2 oder Venus ist.

## Revendications

1. Procédé *in vitro* de criblage d'un promoteur spécifique de cellule indifférenciée, la méthode comprenant les étapes suivantes :
A) remplacement du promoteur d'un vecteur lentiviral contenant une séquence d'acide nucléique dans laquelle un gène de protéine fluorescente et un gène suicide dépendant du médicament sont liés via une séquence capable de permettre à un promoteur d'exprimer simultanément les deux gènes et contenant une cassette de recombinaison ayant la région de promoteur entre des séquences reconnues par des recombinases spécifiques à un site, dans lequel la région de promoteur est liée de manière opérationnelle au gène de la protéine fluorescente et au gène suicide dépendant du médicament par un promoteur de test pour préparer un vecteur lentiviral contenant le promoteur de test ;
B) l'infection des cellules d'intérêt avec le vecteur lentiviral contenant le promoteur de test ;
C) la détection de l'expression du gène de la protéine fluorescente dans lesdites cellules infectées soumises à un traitement d'induction de différenciation ;
D) distinguer si lesdites cellules infectées soumises à un traitement d'induction de différenciation sont des cellules indifférenciées ou des cellules différenciées ; et
E) juger si le promoteur de test est spécifique ou non aux cellules indifférenciées,
dans lequel si le niveau d'expression du gène de la protéine fluorescente dans les cellules indifférenciées est plus élevé que dans les cellules différenciées avec une différence statistique, le promoteur de test est déterminé comme étant spécifique aux cellules indifférenciées.

2. Procédé selon la revendication 1, dans lequel la séquence capable de permettre à un promoteur de provoquer l'expression simultanée des deux gènes est la séquence 2A.

3. Procédé selon la revendication 1 ou 2, dans lequel le gène suicide dépendant du médicament est HSV-tk, tmpk humaine, un gène de cytosine désaminase ou caspase.

4. Procédé selon la revendication 3, dans lequel le gène suicide dépendant du médicament est HSV-tk

5. Procédé selon l'une quelconque des revendications 1 à 4, dans lequel la protéine fluorescente est une protéine fluorescente rouge ou une protéine fluorescente verte.

6. Procédé selon la revendication 5, dans lequel la protéine fluorescente est mKate2 ou Venus.
